# EUROPEAN PATENT APPLICATION

(11) **EP 4 201 430 A1**
(43) Date of publication of application: **28.06.2023**
(21) Application number: 21217492.4
(22) Date of filing: 23.12.2021
(51) Int. Cl.: A61K 47/65, A61K 47/68, C07K 16/00, C12N 9/10, C12N 15/117

(54) **MODIFIED ANTIBODY FOR SITE-SPECIFIC CONJUGATION AND ITS DIAGNOSTIC USE**

(71) Applicant: F. Hoffmann-La Roche AG, 4070 Basel (CH); Roche Diagnostics GmbH, 68305 Mannheim (DE)
(72) Inventor: The designation of the inventor has not yet been filed
(74) Representative: Misera, Simon

(57) **Abstract**

The present invention relates to a modified antibody comprising a heavy chain and a light chain, wherein the antibody is modified to include in one or more of its immunoglobulin polypeptide chains one or more first recognition site(s) for the transglutaminase from *Kutzneria albida* (KalbTG) or a functionally active variant thereof. The one or more first recognition site(s) are introduced at one or more selected position(s) within an antibody's heavy chain and/or an antibody's light chain. The invention further relates to one or more nucleic acid(s) encoding an immunoglobulin polypeptide chain including the one or more recognition site(s), a site-specifically conjugated antibody comprising the modified antibody and one or more labelling domain(s) covalently attached to one or more first recognition sites, a kit for producing the conjugated antibody, a method of specifically labelling the modified antibody by way of site-specific conjugation, the use of the modified antibody for producing a specifically site-specifically conjugated antibody, a method of detecting a target in a sample and the use of the site-specifically conjugated antibody in the detection of a target and/or in the diagnosis.

## Description

The present invention relates to a modified antibody comprising a heavy chain and a light chain, wherein the antibody is modified to include in one or more of its immunoglobulin polypeptide chains one or more first recognition site(s) for the transglutaminase from *Kutzneria albida* (KalbTG) or a functionally active variant thereof. The one or more first recognition site(s) are introduced at one or more selected position(s) within an antibody's heavy chain and/or an antibody's light chain. The invention further relates to one or more nucleic acid(s) encoding an immunoglobulin polypeptide chain including the one or more recognition site(s), a site-specifically conjugated antibody comprising the modified antibody and one or more labelling domain(s) covalently attached to one or more first recognition sites, a kit for producing the conjugated antibody, a method of specifically labelling the modified antibody by way of site-specific conjugation, the use of the modified antibody for producing a specifically site-specifically conjugated antibody, a method of detecting a target in a sample and the use of the site-specifically conjugated antibody in the detection of a target and/or in the diagnosis.

A diagnostic test is a procedure performed to confirm or determine the presence of disease in an individual suspected of having a disease, usually following the report of symptoms, or based on other medical test results. Quantification of a target substance, a cell type or another specific entity is a common output of, for example, most blood tests. This may not only be answering *if* a target entity is present or absent, but also *how much* is present. In blood tests, the quantification is relatively well specified, such as given in mass concentration. The target in question is often detected by use of a target-specific labelled marker.

For this, target-specific antibody-label or antibody-enzyme conjugates are widely used in diagnostic tests. Many of commercially available tests such as Roche Elecsys^{®} assays use conjugated antibodies or polymerized antibodies, with multiple ruthenium labels per antibody, as detection molecules.

Usually, these antibody ruthenium conjugates are currently generated via random targeting of surface lysine residues using succinimide-based chemistry (non-site-specific conjugation). Over the last decade, many technologies for site-specific conjugation have emerged. Collectively, they yield a product with more precise control over the number and insertion site of the labels as well as enhanced homogeneity. These technologies include a plethora of chemical and enzymatic methods targeting: engineered cysteines (e.g. ThioMab), ribosomally-incoporated unnatural amino acids, the antibody-associated carbohydrate chains or a variety of engineered enzyme-recognizable peptide tags (e.g. sortase-, formylglycine-generating-enzyme- or transglutaminase-tags) (Agarwal and Bertozzi, 2015; Zhou and Kim, 2015). Different methods show different sets of advantages, drawbacks and limitations.

However, improved site-specific conjugation technologies remain the target of interest of many medical companies for their potential use in the preparation of therapeutic antibody-drug conjugates as well as diagnostic antibody label or antibody-enzyme conjugates.

Microbial transglutaminase from *Streptomyces mobaraensis* has emerged as an inexpensive and easy to use enzyme for protein crosslinking as well as site-specific protein labelling (Ando et al., 2014). This has been used as well to generate IgGs with drug loading at multiple distinct sites and to show that the site of drug loading onto the IgG greatly affects the performance profile of the antibody-drug conjugate (Strop et al., 2013).

The discovery of a novel transglutaminase (*Kutzneria albida* transglutaminase; KalbTG) and the identification of highly-enzyme-specific peptide substrates have been recently described (Steffen et al., 2017). KalbTG catalyzes the formation of an isopeptide bond between a glutamine side (Gln, Q) chain and a lysine (Lys, K) side chain. YRYRQ (SEQ ID NO: 14) was identified as a KalbTG Gln-containing-motif (Q-tag), and RYESK (SEQ ID NO: 21) was identified as a KalbTG Lys-containing-acceptor-motif (K-tag). KalbTG shows similar efficiency, but improved specificity and developability, compared to other previously described microbial transglutaminases (mTGs).

So far, only the IgG heavy chain C-terminus has been described as a suitable Q-tag insertion site for mTGs for antibody labeling (WO 2021/174091). This limits the application of the method to a labelling rate of two per antibody and devoids it from exploring the label/drug performance dependence on the attachment site. In order to be able to use KalbTG to label Roche Elecsys^{®} detection antibodies with ruthenium/ iridium or in general to prepare antibody-drug/-enzyme conjugates, the KalbTG Q-tag needs to be engineered within the IgG backbone at one or more sites according to the desired labelling rate and preferred labelling site. Internal labelling of antibodies also reduced the mobility of the attached label, which may increase suitability of the labelled antibody.

In order to circumvent the above-mentioned shortcomings, it was an object to identify potential sites within the IgG molecule for the incorporation of recognition sites for the transglutaminase from *Kutzneria albida,* specifically for incorporation of a Q-tag motif. The incorporation should not impair the antibody folding or function and should provide the required accessibility for the enzyme action. The successful identification of such sites would allow the incorporation of not only two, but multiple label molecules per IgG molecule, as the application requires, in a controlled site-specific manner, which provides more homogeneous conjugate products with better lot-to-lot consistency. Moreover, molecules with single insertions at varying sites allow to explore the dependence of the label performance (e.g. signal or blank of ruthenium/iridium labels or pharmacodynamics/kinetic properties of drugs) on the location within the IgG as well as pre-select combinations that provide desired properties (e.g. less ruthenium/iridium label quenching).

The antibody constant domain regions have been screened for conjugation via KalbTG. In this context, KalbTG Q-tags were inserted at surface-exposed inter- and intradomain flexible loops within the human IgG1 constant regions as well as the chains C termini. In total 24 distinct sites within the IgG heavy and light chains were tested; each with an insertion of the KalbTG Q-tag motif within two flexible linkers (GGGSYRYRQGGGS) (SEQ ID NO: 28) and in some instances without the flexible linker flankings (YRYRQ) (SEQ ID NO: 14). These molecules with single-site insertions were assessed for their expression rate, successful folding, stability as well as conjugation efficiency. Insertion sites, which caused a dramatic loss of yield or aggregation, were excluded. Remaining modified antibodies were tested for conjugation and accessibility of the Q-tag for KalbTG to exclude further unsuitable modified antibodies. The inventors have successfully identified a number of KalbTG Q-tag insertion sites spanning the length of the IgG backbone, which provide enzyme accessibility and do not adversely interfere with the IgG cellular expression or folding. Twelve successful insertion sites are at positions position 108 (LC108), 110 (LC110) and 214 (LC214) of the light chain and position 118 (HC118), 177 (HC177), 207 (HC207), 235 (HC235), 269 (HC269), 297 (HC297), 328 (HC328), 341 (HC341) and 401 (HC401) of the heavy chain (the amino acid numbering follows EU-numbering scheme). Additionally, successful combinations of the insertion sites have been identified as well.

Accordingly, in a first aspect the present invention relates to a modified antibody comprising a heavy chain and a light chain, wherein the antibody is modified to include, by way of insertion, one or more first recognition site(s) for the transglutaminase from *Kutzneria albida* (KalbTG), each insertion being located after one or more of the positions selected independently of each other from position 108 (LC108), position 110 (LC110) and position 214 (LC214) of the light chain, and position 118 (HC118), position 177 (HC177), position 207 (HC207), position 235 (HC235), position 269 (HC269), position 297 (HC297), position 328 (HC328), position 341 (HC341) and position 401 (HC401) of the heavy chain. Accordingly, in the modified antibody of the present invention, the recognition site for KalbTG is positioned internally in the constant regions of the Ig heavy chain polypeptide and/or the Ig light chain domain. In addition the modified antibody of the present invention may include a further first recognition site for KalbTG at position 446 (HC446) of the heavy chain, i.e. at the C-terminal end. In the context of the insertion of as recognition site for KalbTG at a position means that the amino acid present at that position in the unmodified sequence is either replaced by the recognition site or is preferably inserted after that position in addition.

The terms " antibody" and "Ig" are used interchangeably herein, and are used in the broadest sense and specifically cover, for example, individual monoclonal antibodies (including agonist, antagonist, neutralizing antibodies, full length or intact monoclonal antibodies), monovalent antibodies, multivalent antibodies, multispecific antibodies (e.g., bispecific so long as they exhibit the desired binding activity and comprise the recognition sites defined above), formed from at least two intact antibodies, single chain antibodies, and fragments of antibodies.

Naturally occurring antibodies are generated by the assembly of heavy chains only or heavy and light chains. Each heavy chain is composed of four domains: the variable domain (VH), CH1, CH2, and CH3. The light chain is composed of variable domain (VL) and constant domain (CL). In case heavy and light chain being present, the light chain pairs with a cognate heavy chain Fab-fragment comprising the VH and CH1 domains. The associated light chain and heavy chain Fab fragment together are denoted as Fab fragment. The heavy chain CH2 and CH3 domains, together denoted as heavy chain Fc-region, dimerize with additional heavy chain CH2 and CH3 domains from a second chain to form the Fc-region. The Fc-region is connected to the Fab-fragment(s) via a flexible hinge region. The hinge region comprises several disulfide bridges that covalently link two heavy chains Fc-regions together. In the Fab-fragment, the light chain and the heavy chains are also connected by one disulfide bridge. However, the connectivity differs among the IgG subclasses. The overall structure of full-length IgGs resembles a Y-shape, with the Fc-region forming the base while the two Fab-fragments form the arms and are available for binding to the antigen.

Within the variable domains reside loops called complementarity determining regions (CDRs). These are mainly responsible for the direct interaction of the antibody with its antigen. Because of the significant variability in the number of amino acids in these CDRs, there are multiple numbering schemes for the variable regions. As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991) and is referred to as "numbering according to Kabat" herein. Specifically, the Kabat numbering system (see pages 647-660) of Kabat, et al., Sequences of Proteins of Immunological Interest, 5th ed., Public Health Service, National Institutes of Health, Bethesda, MD (1991) is used for the light chain constant domain CL of kappa and lambda isotype, and the Kabat EU index numbering system (see pages 661-723) is used for the constant heavy chain domains (CH1, Hinge, CH2 and CH3, which is herein further clarified by referring to "numbering according to Kabat EU index" in this case).

The term "modified antibody" as used herein denotes antibodies or antibody Fc-regions according to the invention comprising at least one (artificial) internal Q-tag at a desired site. The modified antibody is able to bind to a specific antigen and is composed of two pairs of polypeptide chains, wherein each pair has one heavy chain and one light chain and each amino-terminal portion of each chain includes a variable region of about 100 to about 130 or more amino acids and each carboxyl - terminal portion of each chain includes a constant region. Modified antibodies also include, but are not limited to, synthetic antibodies, monoclonal antibodies, recombinant antibodies, multispecific antibodies (including bi-specific antibodies), human antibodies, humanized antibodies, camelized antibodies, chimeric antibodies, intrabodies, anti-idiotypic (anti-id) antibodies, and functional fragments thereof. The term "functional fragment" refers to a portion of an antibody heavy or light chain polypeptide that retains some or all of the binding activity of the modified antibody from which the fragment is derived, with the functional fragment including at least one internal Q-tag. Non-limiting examples of functional fragments of an antibody include single-chain Fvs (scFv) (e.g. including monospecific or bispecific), Fab fragments, F(ab') fragments, F(ab)2 fragments, F(ab')2 fragments, antibody/Fc fusion proteins, disulfide-linked Fvs (sdFv) etc. In particular, modified antibodies provided herein include immunoglobulin molecules and immunologically active portions of immunoglobulin molecules, for example, antigen-binding domains or molecules that contain an antigen-binding site that binds to the antigen (e.g., one or more complementarity determining regions (CDRs)). The antibodies provided herein can be of any type (e.g., IgG, IgE, IgM, IgD, IgA, and IgY, preferably IgG), any class (e.g., IgG1, IgG2, IgG3, IgG4, IgA1, and IgA2), or any subclass (e.g. IgG2a and IgG2b) of an immunoglobulin molecule. An antibody can be human, humanized, chimeric and/or affinity matured as well as an antibody from other species, for example, goat, mouse, rat, rabbit or sheep.

In accordance with the present invention, the modified antibody comprises at least one heavy chain and at least one light chain. Accordingly, the antibody may have one or two Fab domains. In one embodiment, the modified antibody is a monovalent, monospecific antibody comprising one (full-length) light chain and one (full-length) heavy chain forming a cognate light chain-heavy chain-pair (including one binding site) and one heavy chain Fc-region fragment associated with the Fc-region of the heavy chain. In another embodiment, the antibody may have two Fab domains. In such an embodiment, the modified antibody is a bivalent, monospecific antibody comprising two (full-length) light chains and two (full-length) heavy chains forming cognate light chain-heavy chain-pairs (including the antigen binding sites) and a heavy chain Fc-region with the respective paired heavy chain polypeptide domains.

The present invention provides a modified antibody comprising a heavy chain and a light chain, wherein the antibody is modified to include, by way of insertion, one or more first recognition site(s) for the transglutaminase from *Kutzneria albida* (KalbTG), each insertion being located after one or more of the positions selected independently of each other from position 108 (LC108), position 110 (LC110) and position 214 (LC214) of the light chain, and position 118 (HC118), position 177 (HC177), position 207 (HC207), position 235 (HC235), position 269 (HC269), position 297 (HC297), position 328 (HC328), position 341 (HC341) and position 401 (HC401) of the heavy chain. In an embodiment, the modified antibody includes a further first recognition site for KalbTG at position 446 (HC446) of the heavy chain. In accordance with the present invention, the antibody to be modified may be an IgG1, IgG2, IgG3, or IgG4 antibody, particularly a human, mouse, rabbit, or sheep antibody. Exemplary and suitable sequences are given in the following, wherein X^{↓} indicates an insertion site of Q-tag motifs:

Human-Heavy chain-constant (IgG1) (SEQ ID NO. 1) 
A↓STKGPSVFPLAPSSKSTSGGTAALGCLVKDYFPE↓PVTVSWNSGALTSGVHTFPAVLQS
S↓GLYSLSSVVTVPSSSLGTQTYICNVNHKPS↓NTKVDKKVEPKSC↓DKTHTCPPCPAPEL
L↓GGPSVFLFPPKPKDTLMISRTPEVTCVVVDVSHE↓DPEVKFNWYVDGVEVHNAKTKPRE
EQYN↓STYRVVSVLTVLHQDWLNGKEYKCKVSNKAL↓PAPIEKTISKAKG↓QPREPQVYTL
PPSRDELTKNQVSLTCLVKGFYPS↓DIAVEWESNGQPENNYKTTPPVLDSD↓GSFFLYSKL
TVDKSRWQQGNVFSCSVMHEA↓LHNHYTQKSLSLSPG↓
 Human-Heavy chain-constant (IgG2) (SEQ ID NO. 2) 
ASTKGPSVFPLAPCSRSTSESTAALGCLVKDYFPEPVTVSWNSGALTSGVHTFPAVLQS
SGLYSLSSVVTVPSSNFGTQTYTCNVDHKPSNTKVDKTVERKCCVECPPCPAPPVAG
PSVFLFPPKPKDTLMISRTPEVTCVVVDVSHEDPEVQFNWYVDGVEVHNAKTKPREEQFN
STFRVVSVLTVVHQDWLNGKEYKCKVSNKGLPAPIEKTISKTKGQPREPQVYTLPPSR
EEMTKNQVSLTCLVKGFYPSDIAVEWESNGQPENNYKTTPPMLDSDGSFFLYSKLTVDK
SRWQQGNVFSCSVMHEALHNHYTQKSLSLSPG
 Human-Heavy chain-constant (IgG3) (SEQ ID NO. 3) 
A↓STKGPSVFPLAPCSRSTSESTAALGCLVKDYFPE↓PVTVSWNSGALTSGVHTFPAVLQS
S↓GLYSLSSVVTVPSSSLGTKTYTCNVDHKPS↓NTKVDKRVSPNMV↓PHAHHAQAPEFL↓G
GPSVFLFPPKPKDTLMISRTPEVTCVVVDVSQE↓DPEVQFNWYVDGVEVHNAKTKPREEQF
N↓STYRVVSVLTVLHQDWLNGKEYKCKVSNKGL↓PSSIEKTISKAKG↓QPREPQVYTLPPS
QEEMTKNQVSLTCLVKGFYPS↓DIAVEWESNGQPENNYKTTPPVLDSD↓GSFFLYSRLTVD
KSRWQEGNVFSCSVMHEA↓LHNHYTQKSLSLSLG↓
 Human-Heavy chain-constant (IgG4) (SEQ ID NO. 4) 
A↓STKGPSVFPLAPCSRSTSESTAALGCLVKDYFPE↓PVTVSWNSGALTSGVHTFPAVLQS
S↓GLYSLSSVVTVPSSSLGTKTYTCNVDHKPS↓NTKVDKRVSPNMV↓PHAHHAQAPEFL↓G
GPSVFLFPPKPKDTLMISRTPEVTCVVVDVSQE↓DPEVQFNWYVDGVEVHNAKTKPREEQF
N↓STYRVVSVLTVLHQDWLNGKEYKCKVSNKGL↓PSSIEKTISKAKG↓QPREPQVYTLPPS
QEEMTKNQVSLTCLVKGFYPS↓DIAVEWESNGQPENNYKTTPPVLDSD↓GSFFLYSRLTVD
KSRWQEGNVFSCSVMHEA↓LHNHYTQKSLSLSLG↓
 Mouse-Heavy chain-constant (IgG1) (SEQ ID NO. 5) 
A↓KTTPPSVYPLAPGSAAQTNSMVTLGCLVKGYFPE↓PVTVTWNSGSLSSGVHTFPAVLQS
D↓LYTLSSSVTVPSSTWPSETVTCNVAHPAS↓STKVDKKIVPRDC↓GCKPCICTVPEV↓SS
VFIFPPKPKDVLTITLTPKVTCVVVDISKD↓DPEVQFSWFVDDVEVHTAQTQPREEQFN↓S
TFRSVSELPIMHQDWLNGKEFKCRVNSAAF↓PAPIEKTISKTKG↓RPKAPQVYTIPPPKEQ
MAKDKVSLTCMITDFFPE↓DITVEWQWNGQPAENYKNTQPIMDTD↓GSYFVYSKLNVQKSN
WEAGNTFTCSVLHEG↓LHNHHTEKSLSHSPG↓
 Mouse-Heavy chain-constant (IgG2a) (SEQ ID NO. 6) 
A↓KTTAPSVYPLAPVCGDTTGSSVTLGCLVKGYFPE↓PVTLTWNSGSLSSGVHTFPAVLQS
D↓LYTLSSSVTVTSSTWPSQSITCNVAHPAS↓STKVDKKIEPRG↓PTIKPCPPCKCPAPNL
L↓GGPSVFIFPPKIKDVLMISLSPIVTCVVVDVSED↓DPDVQISWFVNNVEVHTAQTQTHR
EDYN↓STLRVVSALPIQHQDWMSGKEFKCKVNNKDL↓PAPIERTISKPKG↓SVRAPQVYVL
PPPEEEMTKKQVTLTCMVTDFMPE↓DIYVEWTNNGKTELNYKNTEPVLDSD↓GSYFMYSKL
RVEKKNWVERNSYSCSVVHEG↓LHNHHTTKSFSRTPG↓
 Mouse-Heavy chain-constant (IgG2b) (SEQ ID NO. 7) 
A↓KTTPPSVYPLAPGCGDTTGSSVTLGCLVKGYFPE↓SVTVTWNSGSLSSSVHTFPALLQS
↓GLYTMSSSVTVPSSTWPSQTVTCSVAHPAS↓STTVDKKLEPSGP↓ISTINPCPPCKECHK
CPAPNLE↓GGPSVFIFPPNIKDVLMISLTPKVTCVVVDVSED↓DPDVRISWFVNNVEVHTA
QTQTHREDYN↓STIRVVSALPIQHQDWMSGKEFKCKVNNKDL↓PSPIERTISKIKG↓LVRA
PQVYILPPPAEQLSRKDVSLTCLVVGFNPG↓DISVEWTSNGHTEENYKDTAPVLDSD↓GSY
FIYSKLDIKTSKWEKTDSFSCNVRHEG↓LKNYYLKKTISRSPG↓
 Rabbit-Heavy chain-constant (SEQ ID NO. 8) 
G↓QPKAPSVFPLAPCCGDTPSSTVTLGCLVKGYLPE↓PVTVTWNSGTLTNGVRTFPSVRQS
S↓GLYSLSSVVSVTSSSQPVTCNVAHPAT↓NTKVDKTVAPSTC↓SKPTCPPPELL↓GGPSV
FIFPPKPKDTLMISRTPEVTCVVVDVSQD↓DPEVQFTWYINNEQVRTARPPLREQQFN↓ST
IRVVSTLPIAHQDWLRGKEFKCKVHNKAL↓PAPIEKTISKARG↓QPLEPKVYTMGPPREEL
SSRSVSLTCMINGFYPS↓DISVEWEKNGKAEDNYKTTPAVLDSD↓GSYFLYNKLSVPTSEW
QRGDVFTCSVMHEA↓LHNHYTQKSISRSPG↓
 Sheep-Heavy chain-constant (SEQ ID NO. 9) 
A↓STTPPKVYPLTSCCGDTSSSIVTLGCLVSSYMPE↓PVTVTWNSGALTSGVHTFPAILQS
S↓GLYSLSSVVTVPASTSGAQTFICNVAHPAS↓STKVDKRVEPGCP↓DPCKHCRCPPPELP
↓GGPSVFIFPPKPKDTLTISGTPEVTCVVVDVGQD↓DPEVQFSWFVDNVEVRTARTKPREE
QFN↓STFRVVSALPIQHQDWTGGKEFKCKVHNEGL↓PAPIVRTISRTKG↓QAREPQVYVLA
PPQEELSKSTLSVTCLVTGFYPD↓YIAVEWQKNGQPESEDKYGTTTSQLDAD↓GSYFLYSR
LRVDKNSWQEGDTYACVVMHEA↓LHNHYTQKSISKPPG↓
 Human-Light chain-constant (SEQ ID NO. 10) 
↓TV↓AAPSVFIFPPSDEQLKS↓GTASVVCLLNNFYPRE↓AKVQWKVDNALQS↓GNSQESVT
EQDS↓KDS↓TYSLSSTLTLSKADYEKH↓KVYACEVTHQGL↓SSPVTKSFNRGEC↓
 Mouse-Light chain-constant (SEQ ID NO. 11) 
ADAAPTVSIFPPSSEQLTSGGASVVCFLNNFYPKDINVKWKIDGSERQNGVLNSWT
DQDSKDSTYSMSSTLTLTKDEYERHNSYTCEATHKTSTSPIVKSFNRNEC
 Rabbit-Light chain-constant (SEQ ID NO. 12) 
DPVAPTVLIFPPAADQVATGTVTIVCVANKYFPDVTVTWEVDGTTQTTGIENSKTP
QNSADCTYNLSSTLTLTSTQYNSHKEYTCKVTQGTTSVVQSFNRGDC
 Sheep-Light chain-constant (SEQ ID NO. 13)

PKSAPSVTLFPPSTEELSTNKATVVCLINDFYPGSVNVVWKADGSTINQNVKTTQA
SKQSNSKYAASSYLTLTGSEWKSKSSYTCEVTHEGSTVTKTVKPSECS

Thus, in some cases, the Ig heavy chain constant region is a human Ig heavy chain constant region, e.g., human IgG1, human IgG2, human IgG3, or human IgG4 heavy chain constant region. In some cases, the heavy chain constant region comprises at least one Q-tag according to the invention.

In some cases, the Ig heavy chain constant region is a mouse Ig heavy chain constant region, e.g., mouse IgG1, mouse IgG2a or mouse IgG2b heavy chain constant region. In some cases, the Ig heavy chain constant region is a rabbit Ig heavy chain constant region or a sheep Ig heavy chain constant region. In some cases, the Ig light chain constant region is a human, mouse, rabbit or sheep Ig light chain constant region.

In a preferred embodiment, (i) the unmodified light chain constant domain (CL) comprises an amino acid sequence that is at least 96 %, 97 %, 98 %, or 99 %, particularly 100 % identical to the amino acid sequence of any of SEQ ID NO: 10 to 13; and/or (ii) any of the unmodified heavy chain constant domains CH3, CH2, and CH1 comprises an amino acid sequence that is at least 96 %, 97 %, 98 %, or 99 %, particularly 100 % identical to the amino acid sequence of the respective domain in the amino acid sequence of SEQ ID NO: 1 to 9; and/or (iii) the unmodified heavy chain constant domain comprises an amino acid sequence that is at least 96 %, 97 %, 98 %, or 99 %, particularly 100 % identical to the respective amino acid sequence of SEQ ID NO: 1 to 9; and/or (vi) the unmodified light chain constant domain (CL) consists of an amino acid sequence that is at least 96 %, 97 %, 98 %, or 99 %, particularly 100 % identical to the amino acid sequence of any of SEQ ID NO: 10 to 13; and/or (v) any of the unmodified heavy chain constant domains CH3, CH2, and CH1 consists of an amino acid sequence that is at least 96 %, 97 %, 98 %, or 99 %, particularly 100 % identical to the amino acid sequence of the respective domain in the amino acid sequence of SEQ ID NO: 1 to 9; and/or (vi) the unmodified heavy chain constant domain consists of an amino acid sequence that is at least 96 %, 97 %, 98 %, or 99 %, particularly 100 % identical to the respective amino acid sequence of SEQ ID NO: 1 to 9.

In some cases, the human IgG1 heavy chain polypeptide, on which the present modified antibody may be based, includes a constant region amino acid sequence 75% or more, e.g., 80% or more, 85% or more, 90% or more, 95% or more, 97% or more, and up to 100% identical to the amino acid sequence set forth in SEQ ID NO:1. In some cases, the human IgG2 heavy chain polypeptide, on which the present modified antibody may be based, includes a constant region amino acid sequence 75% or more, e.g., 80% or more, 85% or more, 90% or more, 95% or more, 97% or more, and up to 100% identical to the amino acid sequence set forth in SEQ ID NO:2. In some cases, the human IgG3 heavy chain polypeptide, on which the present modified antibody may be based, includes a constant region amino acid sequence 75% or more, e.g., 80% or more, 85% or more, 90% or more, 95% or more, 97% or more, and up to 100% identical to the amino acid sequence set forth in SEQ ID NO:3. In some cases, the human IgG4 heavy chain polypeptide, on which the present modified antibody may be based, includes a constant region amino acid sequence 75% or more, e.g., 80% or more, 85% or more, 90% or more, 95% or more, 97% or more, and up to 100% identical to the amino acid sequence set forth in SEQ ID NO:4. In some cases, the mouse IgG1 heavy chain polypeptide, on which the present modified antibody may be based, includes a constant region amino acid sequence 75% or more, e.g., 80% or more, 85% or more, 90% or more, 95% or more, 97% or more, and up to 100% identical to the amino acid sequence set forth in SEQ ID NO:5. In some cases, the mouse IgG2a heavy chain polypeptide, on which the present modified antibody may be based, includes a constant region amino acid sequence 75% or more, e.g., 80% or more, 85% or more, 90% or more, 95% or more, 97% or more, and up to 100% identical to the amino acid sequence set forth in SEQ ID NO:6. In some cases, the mouse IgG2b heavy chain polypeptide, on which the present modified antibody may be based, includes a constant region amino acid sequence 75% or more, e.g., 80% or more, 85% or more, 90% or more, 95% or more, 97% or more, and up to 100% identical to the amino acid sequence set forth in SEQ ID NO:7. In some cases, the rabbit heavy chain polypeptide, on which the present modified antibody may be based, includes a constant region amino acid sequence 75% or more, e.g., 80% or more, 85% or more, 90% or more, 95% or more, 97% or more, and up to 100% identical to the amino acid sequence set forth in SEQ ID NO:8. In some cases, the sheep heavy chain polypeptide, on which the present modified antibody may be based, includes a constant region amino acid sequence 75% or more, e.g., 80% or more, 85% or more, 90% or more, 95% or more, 97% or more, and up to 100% identical to the amino acid sequence set forth in SEQ ID NO:9. In some cases, the human light chain polypeptide, on which the present modified antibody may be based, includes a constant region amino acid sequence 75% or more, e.g., 80% or more, 85% or more, 90% or more, 95% or more, 97% or more, and up to 100% identical to the amino acid sequence set forth in SEQ ID NO:10. In some cases, the mouse light chain polypeptide, on which the present modified antibody may be based, includes a constant region amino acid sequence 75% or more, e.g., 80% or more, 85% or more, 90% or more, 95% or more, 97% or more, and up to 100% identical to the amino acid sequence set forth in SEQ ID NO:11. In some cases, the rabbit light chain polypeptide, on which the present modified antibody may be based, includes a constant region amino acid sequence 75% or more, e.g., 80% or more, 85% or more, 90% or more, 95% or more, 97% or more, and up to 100% identical to the amino acid sequence set forth in SEQ ID NO:12. In some cases, the sheep light chain polypeptide, on which the present modified antibody may be based, includes a constant region amino acid sequence 75% or more, e.g., 80% or more, 85% or more, 90% or more, 95% or more, 97% or more, and up to 100% identical to the amino acid sequence set forth in SEQ ID NO:13. Still more preferably, the unmodified light chain comprises of an amino acid sequence that is at least 96 %, 97 %, 98 %, or 99 %, particularly 100 % identical to the amino acid sequence of any of SEQ ID NO: 10 to 13; and/or wherein the unmodified heavy chain comprises an amino acid sequence that is at least 96 %, 97 %, 98 %, or 99 %, particularly 100 % identical to the amino acid sequence of SEQ ID NO: 1 to 9.

As detailed above, the antibody may be a bi- or multispecific antibody. Exemplary embodiments include:
- full-length antibody with domain exchange:
   a multispecific IgG antibody comprising a first Fab fragment and a second Fab fragment, wherein in the first Fab fragment
      a) only the CH1 and CL domains are replaced by each other (i.e. the light chain of the first Fab fragment comprises a VL and a CH1 domain and the heavy chain of the first Fab fragment comprises a VH and a CL domain);
      b) only the VH and VL domains are replaced by each other (i.e. the light chain of the first Fab fragment comprises a VH and a CL domain and the heavy chain of the first Fab fragment comprises a VL and a CH1 domain); or
      c) the CH1 and CL domains are replaced by each other and the VH and VL domains are replaced by each other (i.e. the light chain of the first Fab fragment comprises a VH and a CH1 domain and the heavy chain of the first Fab fragment comprises a VL and a CL domain); and
   wherein the second Fab fragment comprises a light chain comprising a VL and a CL domain, and a heavy chain comprising a VH and a CH1 domain; the full length antibody with domain exchange may comprises a first heavy chain including a CH3 domain and a second heavy chain including a CH3 domain, wherein both CH3 domains are engineered in a complementary manner by respective amino acid substitutions, in order to support
   heterodimerization of the first heavy chain and the modified second heavy chain;
- full-length antibody with domain exchange and additional heavy chain C-terminal binding site:
   a multispecific IgG antibody comprising
      a) one full length antibody comprising two pairs each of a full length antibody light chain and a full length antibody heavy chain, wherein the binding sites formed by each of the pairs of the full length heavy chain and the full length light chain specifically bind to a first antigen, and
      b) one additional Fab fragment, wherein the additional Fab fragment is fused to the C-terminus of one heavy chain of the full length antibody, wherein the binding site of the additional Fab fragment specifically binds to a second antigen,
   wherein the additional Fab fragment specifically binding to the second antigen i) comprises a domain crossover such that a) the light chain variable domain (VL) and the heavy chain variable domain (VH) are replaced by each other, or b) the light chain constant domain (CL) and the heavy chain constant domain (CH1) are replaced by each other, or ii) is a single chain Fab fragment;
- the one-armed single chain format (= one-armed single chain antibody): antibody comprising a first binding site that specifically binds to a first epitope or antigen and a second binding site that specifically binds to a second epitope or antigen, whereby the individual chains are as follows
   - light chain (variable light chain domain + light chain kappa constant domain)
   - combined light/heavy chain (variable light chain domain + light chain constant domain + peptidic linker + variable heavy chain domain + CH1 + Hinge + CH2 + CH3 with knob mutation)
   - heavy chain (variable heavy chain domain + CH1 + Hinge + CH2 + CH3 with hole mutation);
- the two-armed single chain format (= two-armed single chain antibody): antibody comprising a first binding site that specifically binds to a first epitope or antigen and a second binding site that specifically binds to a second epitope or antigen, whereby the individual chains are as follows
   - combined light/heavy chain 1 (variable light chain domain + light chain constant domain + peptidic linker + variable heavy chain domain + CH1 + Hinge + CH2 + CH3 with hole mutation)
   - combined light/heavy chain 2 (variable light chain domain + light chain constant domain + peptidic linker + variable heavy chain domain + CH1 + Hinge + CH2 + CH3 with knob mutation);
- the common light chain bispecific format (= common light chain bispecific antibody):
   antibody comprising a first binding site that specifically binds to a first epitope or antigen and a second binding site that specifically binds to a second epitope or antigen, whereby the individual chains are as follows
   - light chain (variable light chain domain + light chain constant domain)
   - heavy chain 1 (variable heavy chain domain + CH1 + Hinge + CH2 + CH3 with hole mutation)
   - heavy chain 2 (variable heavy chain domain + CH1 + Hinge + CH2 + CH3 with knob mutation).

As used herein the term "replaced by each other" with respect to corresponding heavy and light chain domains refers to the aforementioned domain crossovers. As such, when CH1 and CL domains are "replaced by each other" it is referred to the domain crossover mentioned under item (i) and the resulting heavy and light chain domain sequence. Accordingly, when VH and VL are "replaced by each other" it is referred to the domain crossover mentioned under item (ii); and when the CH1 and CL domains are "replaced by each other" and the VH and VL domains are "replaced by each other" it is referred to the domain crossover mentioned under item (iii).

Multispecific antibodies also comprise in one embodiment at least one Fab fragment including either a domain crossover of the CH1 and the CL domains as mentioned under item (i) above, or a domain crossover of the VH and the VL domains as mentioned under item (ii) above, or a domain crossover of the VH-CH1 and the VL-VL domains as mentioned under item (iii) above. In case of multispecific antibodies with domain crossover, the Fabs specifically binding to the same antigen(s) are constructed to be of the same domain sequence. Hence, in case more than one Fab with a domain crossover is contained in the multispecific antibody, said Fab(s) specifically bind to the same antigen.

Multivalent IgG derivatives as disclosed in WO2019057816 can be designed having CH1-VH domains fused to either the C- or N-terminus of an IgG heavy chain; together with the associated light chain (CL-VL) they form additional binding sites thus turning a bivalent IgG into tetra- or hexavalent antibody. Further likewise C- or N-terminal additions lead to even longer heavy chain fusion constructs thus permitting even higher degrees of multivalency. Importantly, as the domains in these constructs correspond to the domains with Q-tag insertion sites shown in this document, a multivalent IgG derivative with additional C- or N-terminally appended CH1-VH domains is yet a further embodiment of a modified antibody comprising a heavy chain and a light chain, wherein the antibody is modified to include, by way of insertion, one or more first recognition site(s) for the transglutaminase from *Kutzneria albida* (KalbTG), each insertion being located after one or more of the positions selected independently of each other from position 108 (LC108), position 110 (LC110) and position 214 (LC214) of the light chain, and position 118 (HC118), position 177 (HC177), position 207 (HC207), position 235 (HC235), position 269 (HC269), position 297 (HC297), position 328 (HC328), position 341 (HC341) and position 401 (HC401) of the heavy chain.

In an embodiment the antibody comprises two pairs each of a heavy chain and a light chain, wherein each of the heavy chains or /and the light chains comprises the one or more first recognition sites. In another embodiment the antibody comprises three or more pairs each of a heavy chain and a light chain, wherein each heavy chain is a fusion polypeptide comprising one or more additional Fab domain VH and CH1 heavy chain fragment fused to any of a VH N-terminus and Fc C-terminus of the heavy chain, wherein each of the fusion polypeptides or /and the light chains comprises the one or more first recognition sites. In yet another embodiment the antibody comprises three or more pairs each of a heavy chain and a light chain, wherein each heavy chain is a fusion polypeptide comprising one or more additional Fab domain VL and CL light chain fragment fused to any of a VH N-terminus and Fc C-terminus of the heavy chain, wherein each fused Fab domain VL and CL light chain fragment is paired with a Fab domain VH and CH1 heavy chain fragment, wherein each of the fusion polypeptides or /and the light chains or /and the Fab domain VH and CH1 heavy chain fragment comprises the one or more first recognition sites.

The term "antigen" refers to a predetermined target to which an antibody can selectively bind. An antigen can be a polypeptide, carbohydrate, nucleic acid, lipid, hapten, or fragment thereof, or other naturally occurring or synthetic compound. In one embodiment, the target antigen is a polypeptide. In another embodiment, the target antigen is of diagnostic relevance.

According to the present invention, the modified antibody includes one or more recognition sites for KalbTG in the heavy chain polypeptide and/or in the light chain polypeptide. The recognition site includes a motif for KalbTG, wherein KalbTG can catalyze the formation of an isopeptide bond between the modified antibody and a compound to be coupled to the antibody. Typically, the isopeptide bond is formed between a glutamine side (Gln) chain and a lysine (Lys) side chain. In certain embodiments the Lys side chain can be replaced by a primary amine compound capable of acting as a functional analog of a peptidic KalbTG K-tag. Preferably, the modification of the antibody to create the modified antibody of the present invention includes the generation of a Q-tag, i.e. a Gln containing motif recognized by the KalbTG, in the antibody. Suitable Q-tags are disclosed in WO 2017/102759 A1 and below. The Q-tag may be generated by one or more amino acid modification, such as substitutions or insertions, preferably insertions. The one or more first recognition sites independently of each other comprise or have a Gln-containing motif. More preferably, the Q-tag is generated by insertion and/or replacement of an amino acid sequence comprising any of the sequences of SEQ ID NO: 14 to 20, particularly YRYRQ (SEQ ID NO: 17) or RVRQR (SEQ ID NO: 18), especially YRYRQ (SEQ ID NO: 17). The introduction of the one or more recognition sites for KalbTG may be the only modifications in the constant regions of the light and/or heavy chains. Alternatively further modifications may be present, such as a tag for purification (e.g. a His tag) or other modifications, e.g. for increasing stability or heterodimerization or effector function modification, either alone or in any combination.

More preferably, the Q-tag is generated by insertion of the amino acid sequence comprising YRYRQ (SEQ ID NO: 14) or RVRQR (SEQ ID NO: 15), especially YRYRQ (SEQ ID NO: 14). Preferably, the inserted amino acid sequence has a length of 5 to 20 amino acids and comprises YRYRQ (SEQ ID NO: 14) or RVRQR (SEQ ID NO: 15), especially YRYRQ (SEQ ID NO: 14). In a specific embodiment, the insertion is a Q-tag motif without the linkers, i.e. the insertion is YRYRQ (SEQ ID NO: 14) or RVRQR (SEQ ID NO: 15), particularly (YRYRQ) (SEQ ID NO: 14). The introduction of the one or more recognition sites for KalbTG may be the only modifications in the constant regions of the light and/or heavy chains. Alternatively further modifications may be present, such as a tag for purification (e.g. a His tag) or other modifications, e.g. for increasing stability or heterodimerization or effector function modification, either alone or in any combination.

As stated above, the one or more first recognition site(s) for KalbTG are inserted into the antibody at specific sites, namely at one or more of the positions selected from position position 108 (LC108), position 110 (LC110) and position 214 (LC214) of the light chain, and position 118 (HC118), position 177 (HC177), position 207 (HC207), position 235 (HC235), position 269 (HC269), position 297 (HC297), position 328 (HC328), position 341 (HC341) and position 401 (HC401) of the heavy chain. In addition the modified antibody of the present invention may include a further first recognition site for KalbTG at position 446 (HC446) of the heavy chain, i.e. at the C-terminal end. The amino acid numbering follows the established EU-numbering scheme (Kabat, 1991). If the recognition site is inserted into the amino acid sequence of the light or heavy chain it is inserted immediately following the position specified above. The one or more first recognition site(s) inserted may be 1, 2, 3, 4, 5, 6, 7, 8, 9, 10, 11, 12, 13, 14, 15, 16, 17, 18, 19, 20 or more recognition site(s) inserted. The recognition sites may be inserted into 1, 2, 3 or 4 chains of the antibody. For example, the one or more insertions could be present in
- only one heavy chain, or
- only one light chain, or
- both heavy chains, or
- both light chains, or
- one heavy chain and one light chain, or
- one heavy chain and two light chains, or
- two heavy chains and one light chain, or
- two heavy chains and two light chains.

If two light chains or two heavy chains are modified, the recognition sites inserted may be at different or identical positions with respect to the two chains. For example both heavy chains may have a first recognition site inserted at position 328 (HC328) (identical positions) or one heavy chain has a first recognition site inserted at position 328 (HC328) and the other heavy chain has a first recognition site inserted at position 341 (HC341) (different positions). As detailed above, the presence of multiple first recognition sites in one antibody may be preferred in order to increase number of potential labelling sites in the antibody.

In a preferred embodiment of the present invention, the modified antibody of the first aspect of the present invention includes
a) two first recognition sites for KalbTG, particularly at positions
   - HC118 / HC177; or
   - HC118 / HC328; or
   - HC118 / HC341; or
   - HC118 / HC401; or
   - HC177 / HC207; or
   - HC177 / HC328; or
   - HC177 / HC341; or
   - HC177 / HC401; or
   - HC207 / HC328; or
   - HC207 / HC341; or
   - HC207 / HC401; or
   - HC328 / HC446; or
   - HC341 / HC446; or
   - HC118 / LC214; or
   - HC177 / LC108; or
   - HC177 / LC214; or
   - HC207 / LC214; or
   - HC446 / LC214;
      especially
   - HC118 / HC177; or
   - HC118 / HC328; or
   - HC118 / HC341; or
   - HC177 / HC341; or
   - HC207 / HC341; or
   - HC328 / HC446; or
   - HC341 / HC446; or
   - HC118 / LC214; or
   - HC177 / LC108; or
   - HC177 / LC214; or
   - HC207 / LC214; or
   - HC446 / LC214;
   OR
b) three first recognition sites for KalbTG, particularly at positions
   - HC118 / HC235 / HC341; or
   - HC207 / HC235 / HC341; or
   - HC118 / HC341 / HC446; or
   - HC207 / HC341 / HC446; or
   - HC118 / HC235 / HC328; or
   - HC177 / HC235 / HC328; or
   - HC207 / HC235 / HC328;
   OR
c) four first recognition sites for KalbTG, particularly at positions
   - HC118 / HC235 / HC341 / HC446; or
   - HC207 / HC235 / HC341 / HC446.

As detailed above, the modified antibody includes one or more recognition sites for KalbTG in an immunoglobulin (Ig) heavy chain polypeptide and/or an immunoglobulin (Ig) heavy chain polypeptide. The recognition site includes a motif for KalbTG, wherein KalbTG can catalyze the formation of an isopeptide bond between the modified antibody and a compound to be coupled to the antibody. Typically, the isopeptide bond is formed between a glutamine side (Gln) chain and a lysine (Lys) side chain. However, the K-tag is not entirely restricted to the presence of a Lys moiety as a number of primary amines have been found to be capable of functionally replacing the Lys acceptor site. Preferably, the modification of the antibody to create the modified antibody of the present invention includes the generation of a Q-tag, i.e. a Gln containing motif recognized by the KalbTG, in the antibody. Suitable Q-tags are disclosed in WO 2017/102759 A1. Examples include YRYRQ (SEQ ID NO: 14), YRQRT (SEQ ID NO: 16), RYGQR (SEQ ID NO: 17), RWRQR (SEQ ID NO: 18), RVRQR (SEQ ID NO: 15), IRQRQ (SEQ ID NO: 19) and FRYRQ (SEQ ID NO: 20), particularly YRYRQ (SEQ ID NO: 14). The Q-tag may be generated by one or more amino acid modification, such as substitutions or insertions, preferably insertions. The one or more first recognition sites independently of each other comprise or have a Gln-containing motif. More preferably, the Q-tag is generated by insertion and/or replacement of an amino acid sequence comprising any of the sequences of SEQ ID NO: 14 to 20, particularly YRYRQ (SEQ ID NO: 14) or RVRQR (SEQ ID NO: 15), especially YRYRQ (SEQ ID NO: 14). Preferably, the inserted amino acid sequence has a length of 5 to 20 amino acids and comprises YRYRQ (SEQ ID NO: 14) or RVRQR (SEQ ID NO: 15), especially YRYRQ (SEQ ID NO: 14). In a specific embodiment, the insertion is a Q-tag motif without the linkers, i.e. the insertion is YRYRQ (SEQ ID NO: 14) or RVRQR (SEQ ID NO: 15), particularly (YRYRQ) (SEQ ID NO: 14).

In a preferred embodiment, the Q-tag is inserted into the antibody light/heavy chain amino acid sequence via one or two linker(s). The linkers may increase flexibility or allow for coupling of larger labels. Preferably, the linker sequence comprises between 1 to 20 amino acids, preferably 1 to 10 amino acids, more preferred 1 to 5 amino acids, and more preferred said amino acids do not interfere essentially with the function of the Q-tag, the KalbTG, the folding of the antibody and the label to be attached to the modified antibody. The linker may be N- and/or C-terminally attached to the Q-tag. Preferred linker amino acids are small amino acids, like glycine or alanine. Amino acid linkers and their compositions are known in the art (see, e.g. Chichili et al. Linkers in the structural biology of protein-protein interactions Protein Sci. 2013 Feb; 22(2): 153-167). Amino acids glycine, serine, alanine, threonine and glutamate usually constitute amino acids of flexible linkers. Accordingly, the linker(s) may consist primarily or fully of Gly and/or Ser and/or Ala and/or Thr and/or Glu, such as GGGP, ESGS or APAP. Also, a linker comprising or consisting of KESGSVSSEQLAQFRSLD (SEQ ID NO: 26) and EGKSSGSGSESKST (SEQ ID NO: 27) may be present. Especially preferred are linkers consisting primarily or fully of Gly and Ser, such as (GlyₘSer)ₙ with m = 1, 2, 3 or 4 and n = 1, 2, 3, 4 or 5, m and n being independently of each other, preferably m=3 and n=1. Preferably, the first recognition site or any one of two or more first recognition sites is linked to the antibody via one or two linker(s) at one or both termini of the respective first recognition site, especially wherein the linker consists primarily or fully of Gly and Ser, such as (Gly-Gly-Gly-Ser)ₙ (SEQ ID NO: 22) with n = 1, 2, 3, 4 or 5, preferably n=1, or especially wherein the linker comprises or consists of GGGP (SEQ ID NO: 23), ESGS (SEQ ID NO: 24) or APAP (SEQ ID NO: 25). In embodiments with a plurality of linkers, the amino acid sequence of each linker is selected independently.

If a linker is present, the insertion of the amino acid sequence X1-YRYRQ-X2 (SEQ ID NO: 14) or X1-RVRQR-X2 (SEQ ID NO: 15) into the antibody is preferred. X1 and X2 are independently from each other absent or a linker, particularly linker amino acids. In a specific embodiment, the insertion is a Q-tag motif with two flexible linkers, particularly (GGGSYRYRQGGGS) (SEQ ID NO: 28) or GGGSRVRQRGGGS (SEQ ID NO: 29), especially GGGSYRYRQGGGS) (SEQ ID NO: 28).

In a highly preferred embodiment, the light chain constant domain comprises or consists of an amino acid sequence that is at least 96 %, 97 %, 98 %, or 99 %, particularly 100 % identical to the amino acid sequence of any of SEQ ID NO: 10 to 13; and/or the heavy chain constant domain comprises or consists of an amino acid sequence that is at least 96 %, 97 %, 98 %, or 99 %, particularly 100 % identical to the amino acid sequence of SEQ ID NO: 1 to 9.

In a second aspect, the present invention relates to a nucleic acid coding for a polypeptide chain of the modified antibody of the present invention.

The nucleic acid encoding the modified antibody of the present invention can be isolated or generated in vitro to produce the antibody recombinantly. The nucleic acid may be isolated and inserted into a replicable vector for further cloning (amplification of DNA) or for further expression.

"Nucleic acid" has the meaning of comprehensively including DNA (gDNA and cDNA) and RNA molecules, and nucleotides, which are basic structural units in nucleic acids, include not only natural nucleotides, but also analogs with modified sugar or base sites. The sequence of the nucleic acid encoding the heavy and light chain variable regions of the present invention can be modified. Such modifications include additions, deletions, or non-conservative or conservative substitutions of nucleotides as long as the encoded sequence is not changed.

The DNA encoding the antibody is easily isolated or synthesized using conventional procedures (eg, by using an oligonucleotide probe capable of specifically binding to DNA encoding the heavy and light chains of the antibody). Many vectors are available. Vector components generally include, but are not limited to, one or more of the following: signal sequence, origin of replication, one or more marker genes, enhancer elements, promoters, and transcription termination sequences.

The term "vector" as used herein refers to a plasmid vector as a means for expressing a gene of interest in a host cell; Cozmid vector; viral vectors such as bacteriophage vectors, adenovirus vectors, retroviral vectors and adeno-associated viral vectors, and the like. The nucleic acid encoding the modified antibody in the vector is operably linked to a promoter.

"Operatively linked" refers to a functional linkage between a nucleic acid expression control sequence (eg, a promoter, a signal sequence, or an array of transcriptional regulatory factor binding sites) and another nucleic acid sequence, whereby the control sequence is the other nucleic acid Control the transcription and/or translation of the sequence.

In the case of using prokaryotic cells as a host, a strong promoter capable of promoting transcription (e.g., tac promoter, lac promoter, lacUV5 promoter, Ipp promoter, pLλ promoter, pRλ promoter, rac5 promoter, amp promoter, recA promoter, SP6 promoter, trp promoter and T7 promoter, etc.), ribosome binding sites for initiation of translation, and transcription/translation termination sequences. In addition, for example, in the case of eukaryotic cells as a host, promoters derived from the genome of mammalian cells (eg, metallotionine promoter, β-actin promoter, human hemoglobin promoter, and human muscle creatine promoter) or mammalian Promoters derived from animal viruses (e.g., adenovirus late promoter, vaccinia virus 7.5K promoter, SV40 promoter, cytomegalovirus (CMV) promoter, tk promoter of HSV, mouse mammary tumor virus (MMTV) promoter, LTR promoter of HIV), Moloney virus promoter Epstein-Barr virus (EBV) promoter and Rous sarcoma virus

(RSV) promoter) may be used, and generally have a polyadenylation sequence as a transcription termination sequence.

In some cases, the vector may be fused with other sequences to facilitate purification of the antibody expressed therefrom. Sequences to be fused include, for example, glutathione S-transferase (Pharmacia, USA), maltose binding protein (NEB, USA), FLAG (IBI, USA) and 6x His (hexahistidine; Quiagen, USA).

The vector contains an antibiotic resistance gene commonly used in the art as a selection marker, for example, for ampicillin, gentamicin, carbenicillin, chloramphenicol, streptomycin, kanamycin, geneticin, neomycin, and tetracycline. There is a resistance gene.

Cells may be transformed with the aforementioned vectors. The cells used to generate the antibodies of the present invention may be prokaryotic, yeast or higher eukaryotic cells, but are not limited thereto.

Strains of the genus Bacillus such as Escherichia coli, Bacillus subtilis and Bacillus thuringensis, Streptomyces, Pseudomonas (e.g., Pseudomonas putida), Proteus Prokaryotic host cells such as Proteus mirabilis and Staphylococcus (eg, Staphylocus carnosus) can be used.

However, the greatest interest in animal cells and examples of useful host cell lines are COS-7, BHK, CHO, CHO-S, CHOK1, GS-CHO, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6 , SP2/0, NS-0, U20S, or HT1080, but is not limited thereto.

In a third aspect, the present invention relates to a site-specifically conjugated antibody, wherein the site-specifically conjugated antibody comprises the modified antibody of the present invention (as defined above) and
(i) one or more labelling domain(s), wherein each of the one or more labelling domain(s) comprises a second recognition site for KalbTG or a functionally active variant thereof, wherein the second recognition site is covalently attached to a first recognition site(s) for KalbTG or a functionally active variant thereof which is present in the modified antibody, and wherein the labelling domain comprises a detectable label; or
(ii) one or more chemical linker moiety /moieties, wherein each of the one or more covalently attached chemical linker moiety /moieties comprises a second recognition site for KalbTG or a functionally active variant thereof, wherein the second recognition site is covalently attached to a first recognition site of the antibody, and wherein the chemical linker moiety comprises a functional group capable of forming a covalent bond in a chemical (non-enzymatic) coupling reaction, particularly a functional group with a reaction partner of carbodiimide or N-hydroxy succinimide coupling chemistry, or a functional group with a reaction partner of so-called "click" chemistry, specifically a functional group with an azide or alkyne moiety.

In this regard, "Click" chemistry refers to a group of reactions that are fast, simple to use, easy to purify, versatile, regiospecific, and give high product yields. While there are a number of reactions that fulfill the criteria, the Huisgen 1,3-dipolar cycloaddition of azides and terminal alkynes, especially cyclic alkynes are widely used. "Click" chemistry applications exist in a wide variety of research areas, including materials sciences, polymer chemistry, and especially in biochemistry and pharmaceutical sciences. In a specific embodiment, a chemical linker moiety comprises an alkyne group or an azide group which can be used as a reactive partner in a "click" reaction. Among the known varieties of "click" chemistry, the Cu(I)-catalyzed Huisgen 1,3-dipolar cycloaddition of azides and terminal alkynes to form 1,2,3-triazoles is one of the most frequently reported examples. This reaction exclusively forms 1,4-substituted products, making it regiospecific. It typically does not require temperature elevation but can be performed over a wide range of temperatures (0-160°C), in a variety of solvents (including water), and over a wide range of pH values (5 through 12). It proceeds as much as 107 times faster than the uncatalyzed version, and purification is typically easy to perform. Furthermore, the reaction appears to be unaffected by steric factors, i.e. substituents which are attached to the a reactive partners in the "click" reaction. All of these characteristics make this cycloaddition particularly popular among the other click reactions described above.

In an embodiment the functional group with a reaction partner of so-called "click" chemistry is an azide group with which a "click" reaction can be performed with an alkynes or a phosphine as reaction. Especially cyclooctyne derivatives are known to the person of skill for the modification of biomolecules under mild conditions (WO 2006/050262). In a specific embodiment in. Thus, the site-specifically conjugated antibody comprises a covalently attached (via the K-tag or functional equivalent thereof) chemical linker moiety with (i) an alkyne or (ii) an azide reaction partner capable of performing a "click" reaction with a respective "click" reaction partner.

As detailed above, the modified antibody of the present invention is provided for coupling one or more labelling domain(s) to specific one or more internal site(s) of the antibody with the aid of KalbTG, thus creating specifically labelled antibodies, which may be used in the detection of targets of interest. The modified antibody is capable of binding to a target antigen of interest and is detected via the one or more labels attached thereto. Thereby, the target of interest may be detected and/or quantified.

Label detection methods are well-known and frequently used in the field of in vitro diagnosis. Exemplary methods include fluorescent labelling, isotopic labelling, chemiluminescent labelling, chemiluminescent labelling, and nanoparticle labelling. Fluorescent label detection methods are the most common and convenient techniques to transmit information from molecular events. Fluorescence probes are stable, easily manipulated, and provide good sensitivity and resolution. In an antibody sandwich assay system, a fluorescent dye molecule is used as a secondary antibody label. In this way, one can omit a direct non-native effect on the molecule of interest. Fluorescence labels are currently available in many forms, from quantum dots and small organic molecules to fluorescent proteins with a range of brightness and specificity that can be selected based on need. The emergence of advanced fluorescence techniques such as fluorescence resonance energy transfer (FRET), bimolecular fluorescence complementation (BiFC), and fluorescence correlation spectroscopy (FCS) has enabled the use of smaller samples with lower detection limits. Exemplary labels include fluorescein, rhodamine and cyanine derivatives. One of the earliest methods for molecular detection involved the use of radioisotopes that enables accurate quantification of targets. Although radioactivity suitable due to sensitivity and specificity, as well as the possibility of fluorescence detection, the use of isotope-labelled molecules raises some safety concerns. However, used labels include ³H, ³²P, ³⁵S and ¹²⁵I. Chemiluminescence is another phenomenon that is useful to transduce molecular interactions into analyzable color information. Obtaining its energy from exoergic reactions, different wavelengths are emitted during molecular relaxation to its ground state depending on the amount of energy acquired. The heterogenous method is the more widely used chemiluminescent assay. Chemiluminescent methods can be direct - using luminophore markers - or indirect - using enzyme markers. In direct chemiluminescent methods, the luminophore labels used are typically acridinium and ruthenium esters, while the enzymatic labels used in indirect methods include alkaline phosphatase with adamantyl 1, 2-dioxetane aryl phosphate (AMPPD) substrate and horseradish peroxidase with luminol or its derivatives as substrate. The detectable label may also be an indirect label, wherein its visualization requires a secondary reporter molecule. The skilled person is moreover well aware of binding pairs consisting of a first and a second binding partner, wherein the binding pair can be formed by specific non-covalent interaction of the first binding partner with the second binding partner. Well-known exemplary binding pairs comprising such a first and second binding partner are selected from the group consisting of
- an antigen and an antigen-specific antibody;
- a hapten and a hapten-specific antibody;
- a ligand and a specific ligand-binding domain;
- an oligo- or polysaccharide and a lectin, the lectin being capable of specifically binding to the oligo- or polysaccharide;
- a histidine-tag and a metal-chelate complex comprising a metal ion selected from Zn²⁺, Ni²⁺, Co²⁺, and Cu²⁺, the metal-chelate complex being capable of binding to the histidine-tag;
- an indium chelate complex and the CHA255 antibody;
- a cucurbit[n]uril host residue and a guest residue capable of binding the host residue;
- a first and a second protein dimerization domain, optionally in the presence of a dimerization inducing or enhancing agent; and
- a first and a second oligonucleotide spiegelmer, each consisting of L-ribose- or L-2'-deoxyribose-containing nucleoside monomers, the first oligonucleotide spiegelmer being capable of hybridizing with the second oligonucleotide spiegelmer;
- a first and a second oligomer consisting of beta-L-LNA nucleoside monomers, the first oligomer being capable of hybridizing with the second oligomer. Thus, the label can generally be a single-stranded oligonucleotide or analog thereof. However, single-stranded all-L-LNA oligomers as described in WO2019/243391 and WO2020/245377 are particularly advantageous as labels. A conjugated single-stranded L-LNA oligomer as label can be bound by a further single-stranded L-LNA oligomer with a complementary sequence and capable of hybridizing with the label oligomer. further single-stranded L-LNA oligomer in an exemplary case is coupled to a moiety which is capable of generating a detectable signal.

Biotin, also known as vitamin H, is often used as a label. Biotin can be detected and visualized with the aid of the highly efficient Biotin binder Streptavidin. Biotinylated compounds are therefore easily detected through Streptavidin carrying either a reporter enzyme or a fluorescent dye. Since however, endogenous biotin from biological samples often interferes with biotin detection, the use of Digoxigenin, a steroid exclusively present in Digitalis plants, may sometimes be preferable. Visualization of incorporated Digoxigenin is achieved in a similar manner by Digoxigenin antibody conjugates with a reporter enzyme or a fluorescent dye.

The choice of label and method of detection is generally determined by the particular downstream application. While speed of detection and ease of quantification are major advantages of fluorescent labels, some indirect methods with secondary reporter molecules allow signal amplification with resultant increased sensitivity. The above methods may be competitive or non-competitive and include sandwich immunoassays and competitive immunoassays.

In a highly preferred method, the site-specifically conjugated antibody is labelled with a label allowing for electrochemiluminescence detection such as luminol, an acridinium ester, a ruthenium ester, an iridium ester or, especially a ruthenium or iridium ester. Particularly, a label is covalently attached to a chemical linker, wherein the label is selected from any of a biotin moiety, a fluorescent dye, an iridium label, a ruthenium label, a radiolabel, a single-stranded oligonucleotide or analog thereof, and a chemiluminescent label.

In a preferred embodiment,
a) the label or the labelling domain is suitable for detection by means of an enzyme-mediated reaction, such as enzyme-mediated chromogenic, fluorogenic or metallographic reaction, or by means of direct or indirect fluorescence analysis or by means of radioanalysis or by means of electrochemoluminescence; and/or
b) the label or the labelling domain is or comprises an enzyme, an enzyme substrate, a chromophore, a fluorophore, a quencher, a radiolabel, a biotin, a metal, or a electrochemiluminescent moiety, specifically an electrochemiluminescent moiety comprising iridium or ruthenium; and/or
c) each site-specifically conjugated antibody is labelled with at least 1, at least 2, at least 3, at least 4, at least 5, at least 6, at least 7 or at least 8 labelling domains, particularly with at least 2, at least 4, at least 6 or at least 8 labels or labelling domains.

These labels and detection methods are well known in the art.

In an embodiment of the site-specifically conjugated antibody of the invention, the linker is a mono or bi-functional chemical linker moiety permitting covalent attachment of one or two reactive further compounds, respectively. The conjugate refers to a compound formed by covalently joining of two or more compounds. Two or more compounds can be covalently joined by a bifunctional linker, wherein a first covalent bond is formed between a first reactive group of the linker and the first chemical compound, and a second covalent bond is formed between the second reactive group of the linker and the second chemical compound.

In a fourth aspect, the present invention provides a kit for producing a site-specifically conjugated antibody, the kit comprising the modified antibody of the present invention (as defined above) and
(i) KalbTG or a functionally active variant thereof;
(ii) a labelling domain capable of being bound to the one or more first recognition site(s) by KalbTG or a functionally active variant thereof, wherein the labelling domain comprises a detectable label; and
(iii) a chemical linker moiety capable of being bound to the one or more first recognition site(s) by KalbTG or the functionally active variant thereof, wherein the chemical linker moiety comprises a functional group functional group capable of forming a covalent bond in a chemical (non-enzymatic) coupling reaction, particularly a functional group with a reaction partner of carbodiimide or N-hydroxy succinimide coupling chemistry, or a functional group with a reaction partner of so-called "click" chemistry, specifically a functional group with an azide or alkyne moiety.

Microbial transglutaminases (MTGs) including KalbTG catalyze the formation of Gln-Lys isopeptide bonds and are widely used for the cross-linking of proteins and peptides in food and biotechnological applications (e.g. to improve the texture of protein-rich foods or in generating antibody-drug conjugates). KalbTG exhibits essentially no cross-reactivity with known MTG substrates or commonly used target proteins, such as antibodies and therefore allows for the specific labelling at predetermined sites. Accordingly, essentially any labelling domain comprising a detectable label and second recognition site for KalbTG, especially wherein the second recognition site comprises or has a Lys-containing motif (K-tag), especially the sequence RYESK (SEQ ID NO: 21) may be coupled to the modified antibody at the one or more first recognition site(s). KalbTG or a functionally active variant thereof may be as defined in Steffen at al., 2017 or WO 2016/100735 A1. The functionally active variant may be a transglutaminase having at least 80%, 90%, 95% or 100% sequence identity to the KalbTG of WO 2016/100735 A1 (see SEQ ID NO:6). Alternatively, the KalbTG or a functionally active variant thereof may be part of a fusion protein additionally comprising a label such as a tag, e.g. for purification purposes.

The labelling domain comprises a detectable label and a second recognition site for KalbTG. The detectable label may be any label defined above, e.g. in the context of the third aspect of the present invention. Suitable labels include a biotin moiety, a fluorescent dye, an iridium label, a ruthenium label, a radiolabel, a single-stranded oligonucleotide or analog thereof, and a chemiluminescent label. For the purpose of this disclosure it is understood that the detectable label forming part of the labelling domain does not shield the second recognition site for KalbTG from access to the active center of KalbTG and also permits contact of the second recognition site with the first recognition site. Thus, the second recognition site may optionally be connected to the detectable label via a linker which covalently couples detectable label and the second recognition site.

In a fifth aspect, the present invention provides a method of site-specifically conjugating a modified antibody, the method comprising
a) providing the modified antibody of the present invention (as defined above),
b) providing a labelling domain, wherein the labelling domain comprises (i) a detectable label, (ii) a second recognition site for KalbTG, especially wherein the second recognition site comprises or has a motive with a primary amine group, more specifically a Lys-containing motif (K-tag), especially the sequence RYESK (SEQ ID NO: 21), and (iii) optionally a linker between the label and the second recognition site; and
c) reacting the modified antibody of a) and the labelling domain of b) in the presence of KalbTG or a functionally active variant thereof and under conditions conducive to the activity of KalbTG, thereby forming a isopeptide bond between the first and the second recognition site, thus site-specifically conjugating the modified antibody,
or
a) providing the modified antibody of the present invention (as defined above),
b) providing a chemical linker moiety, wherein the chemical linker moiety comprises (i) a functional group functional group capable of forming a covalent bond in a chemical (non-enzymatic) coupling reaction, particularly a functional group with a reaction partner of carbodiimide or N-hydroxy succinimide coupling chemistry, or a functional group with a reaction partner of so-called "click" chemistry, specifically a functional group with an azide or alkyne moiety, and (ii) a second recognition site for KalbTG, especially wherein the second recognition site comprises or has a motive with a primary amine group, more specifically a Lys-containing motif, especially the sequence RYESK (SEQ ID NO: 21); and
c) reacting the modified antibody of a) and the chemical linker moiety of b) in the presence of KalbTG or a functionally active variant thereof and under conditions conducive to the activity of KalbTG, thereby forming a isopeptide bond between the first and the second recognition site, thus site-specifically conjugating the modified antibody.

In the method, the modified antibody of the present invention and the labelling domain are provided. In a first alternative, the labelling domain comprises (i) a detectable label, (ii) a second recognition site for KalbTG, especially wherein the second recognition site comprises or has a Lys-containing motif (K-tag), especially the sequence RYESK (SEQ ID NO: 21). The labelling domain may be as defined above. The modified antibody and the labelling domain are reacted in the presence of KalbTG or a functionally active variant thereof and under conditions conducive to the activity of KalbTG, thereby forming an isopeptide bond between the first and the second recognition site, thus specifically labelling the modified antibody. Preferably, in this method according to the present invention, the antibody comprises one or more Q-tag(s), which is/are labelled using the activity of KalbTG. Exemplarily, the label to be attached is selected from an enzyme, biotin, a radioactive group, a dye, such as a fluorescent dye, an isotope, and a metal. The labelling domain also comprises a second recognition site for KalbTG. Preferably, the labelling domain comprises a K-tag having at least 80% sequence identity to the peptide sequence RYESK (SEQ ID NO: 21). In a second alternative, the chemical linker moiety comprises (i) a functional group functional group capable of forming a covalent bond in a chemical (non-enzymatic) coupling reaction, particularly a functional group with a reaction partner of carbodiimide or N-hydroxy succinimide coupling chemistry, or a functional group with a reaction partner of so-called "click" chemistry, specifically a functional group with an azide or alkyne moiety, and (ii) a second recognition site for KalbTG, especially wherein the second recognition site comprises or has a motive with a primary amine group, more specifically a Lys-containing motif, especially the sequence RYESK (SEQ ID NO: 21). The chemical linker moiety may be as defined above. The modified antibody and the chemical linker moiety are reacted in the presence of KalbTG or a functionally active variant thereof and under conditions conducive to the activity of KalbTG, thereby forming an isopeptide bond between the first and the second recognition site, thus site-specifically conjugating the modified antibody. Preferably, in this method according to the present invention, the antibody comprises one or more Q-tag(s), which is/are labelled using the activity of KalbTG. The labelling domain also comprises a second recognition site for KalbTG. Preferably, the chemical linker moiety comprises a K-tag having at least 80% sequence identity to the peptide sequence RYESK (SEQ ID NO: 21).

Preferred is a method according to the present invention, wherein said labelling is controlled and, for example, achieved in a stoichiometric ratio of label and antibody, for example at about 1:1. In another embodiment the controlled reaction takes into account the number of first recognition sites per antibody, and the stoichiometric ratio of label and antibody. Thus, multiple labelling can be achieved and is preferred using more than one first recognition sites on one antibody in order to attach two or even multiple labels to the antibody. The present invention is of particular use for immunological reactions and assays, where quantification is desired.

In a sixth aspect, the present invention relates to the use of the modified antibody of the present invention (as defined above), KalbTG or a functionally active variant thereof, and (i) a second recognition site for KalbTG coupled to a detectable label or (ii) a second recognition site for KalbTG coupled to a chemical linker moiety which comprises a functional group capable of forming a covalent bond in a chemical coupling reaction, for producing a site-specifically conjugated antibody. The definitions and comments with respect to the fifth aspect apply.

In a seventh aspect, the present invention relates method of detecting a target in a sample, the method comprising
a) contacting the sample, suspected of comprising the target, with the site-specifically conjugated antibody of the present invention (as defined above), wherein the site-specifically conjugated antibody is capable of specifically binding to the target, under conditions conducive to the binding of the antibody to the target;
b) optionally removing unbound site-specifically conjugated antibody; and
c) detecting the label of the site-specifically conjugated antibody specifically bound to the target, thereby detecting the target.

In a first step a sample is provided and contacted with the site-specifically conjugated antibody of the present invention. The sample may be any sample suspected of containing a target of interest, including a sample from a subject. A sample is a limited quantity of material which is intended to be identical to and represent a larger amount of that material(s). An act of obtaining a sample can be done by a person or automatically. Samples can be taken or provided for testing, analysis, inspection, investigation, demonstration, or trial use. Sometimes, sampling may be continuously ongoing. The sample may comprise or consist of a solid, a liquid or a gas; it may be material of some intermediate characteristics such as gel or sputum, tissue, organisms, or a combination of these. Preferably, the sample is liquid or a suspension which allows for easy distribution.

The sample in the present context is a quantity of material that is suspected of containing one or more target(s) that are to be detected or measured and quantified. As used herein, the term includes - without limitation - a specimen (e.g., a biopsy or medical specimen), a culture (e.g., microbiological culture) or an environmental sample, such as water or soil. Samples may be from a subject, such as an animal or human, they may be fluid, solid (e.g., stool), a suspension or tissue. The term "sample from a subject" includes all biological fluids, excretions and tissues isolated from any given subject. Preferably, the subject is an animal, more preferably a mammal or still more preferably a human. The sample may be obtained from all of the various families of domestic animals, as well as feral or wild animals, including, but not limited to, such animals as ungulates, bear, fish, rodents, etc.

Examples of samples include, but are not limited to, cell or tissue cultures, blood, blood serum, blood plasma, needle aspirate, urine, semen, seminal fluid, seminal plasma, prostatic fluid, excreta, tears, saliva, sweat, biopsy, ascites, cerebrospinal fluid, pleural fluid, amniotic fluid, peritoneal fluid, interstitial fluid, sputum, milk, lymph, bronchial and other lavage samples, or tissue extract samples The source of the sample may be solid tissue as from a fresh, frozen and/or preserved organ or tissue sample or biopsy or aspirate; or cells from any time in gestation or development of the subject.

The sample may contain compounds which are not naturally intermixed with the source of the sample in nature such as preservatives, anticoagulants, buffers, fixatives, nutrients, antibiotics, or the like.

The target of interest may be indicative of a microorganism (such as a pathogen) and may be useful in the diagnosis of a disease, such as an infection. Infections may be caused by bacteria, viruses, fungi, and parasites or other nucleic acids containing objects. The pathogen may be exogenous (acquired from environmental or animal sources or from other persons) or endogenous (from the normal flora). Samples may be selected on the basis of signs and symptoms, should be representative of the disease process, and should be collected before administration of antimicrobial agents. The amount of the nucleic acid in the unprocessed sample may be indicative of the severity of the disease.

Alternatively, the target may be indicative of a disease or disorder. A disorder relates to a disordered or incorrectly functioning organ, part, structure, or system of the body resulting from the effect of genetic or developmental errors, infection, poisons, nutritional deficiency or imbalance, toxicity, or unfavorable environmental factors. It may be an illness of a human, an animal or plant, caused by infection or a failure of health. Therefore are many markers (such as blood markers) being indicative for a disease state.

The sample, suspected of comprising the target, is contacted with the site-specifically conjugated antibody of the present invention (as defined above), wherein the site-specifically conjugated antibody is capable of specifically binding to the target, under conditions conducive to the binding of the antibody to the target. The antibody recognizes the target and binds thereto with the complementarity determining regions (CDRs) formed by one or more variable regions of an antibody. "Bind" as used in reference to an antibody may refer to the physical interaction between an antibody and a target (e.g., an antigen) characterized by an affinity (K_{D}) value of 10⁻⁶ M or less, e.g., 10⁻⁷ M or less, 10⁻⁸ M or less, 10⁻⁹ M or less, 10⁻¹⁰ M or less, including 10⁻¹¹ M or less. A lower K_{D} value corresponds to a higher binding affinity (i.e., stronger binding) so that a K_{D} value of 10⁻⁷ M indicates a higher binding affinity than a K_{D} value of 10⁻⁶ M. After the contacting and binding, unbound site-specifically conjugated antibody may be removed, e.g. by a washing step. Then, the label(s) of the site-specifically conjugated antibody specifically bound to the target are detected, thereby detecting the target. The detection method evidently depends from the nature of the detectable label. In the methods of the present invention, the presence, amount or concentration of target may be determined. The detection of the presence or changed amount relative to a control is indicative for, e.g. a disease, the presence of a microorganism, or a state.

In methods which employ electrochemoluminescence (such as immunodetection using Roche Elecsys^{®} analyzers), the antibody may be labelled with a luminophore such as a ruthenium ester (e.g. [Ru(bpy)₃]²⁺, tris(2,2'-bipyridine)ruthenium(II) (Ru²⁺)). In the presence of a target, the site-specifically conjugated antibody with the label is immobilized on the working electrode (e.g. via magnetic beads on a second antibody binding the target; sandwich assay). A coreactant (e.g. oxalate ion (C₂O₄²⁻), peroxydisulfate (persulfate, S2O82-), tri-n-propylamine (TPrA) and other amine-related derivatives, and hydrogen peroxide (H₂O₂)) is oxidized at the electrode, generating the radical cation, which rapidly deprotonates, forming the radical. The radical and radical cation react with the luminophore (e.g. the ruthenium ester), which emits photons, which are detected.

In a preferred embodiment, the target is a protein, such as a protein indicative of a microorganism, a cell, especially a tumor cell, a virus, a bacterium, a fungus, a mammal species, a genetic status, a physiological status or a disease, or another antibody.

In another preferred embodiment, the sample has been obtained from a source suspected of being contaminated, a cell culture or a subject, particularly wherein the subject is selected from the group consisting of a human, an animal and a plant, especially a human.

In still another embodiment, the sample is selected from the group consisting of a body fluid, blood, blood plasma, blood serum, urine, bile, cerebrospinal fluid, synovial fluid, a swab, a clinical specimen, an organ sample and a tissue sample.

The methods of the invention are of particular interest in the medical field such as in diagnosis or in therapeutic monitoring and may be used in order to detect and/or quantify a target of interest indicative of a specific microorganism, cell, virus, bacterium, fungus, mammal species, genetic status or a disease. In accordance with this, the methods may be used in the detection of a pathogen. A pathogen has the potential to cause a disease. Typically pathogen is used to describe an infectious agent such as a virus, bacterium, prion, a fungus, or even another microorganism. Of cause, the methods of the invention may also be used to detect non-pathogenic microorganisms.

Exemplary pathogens include without limitation:
- Bacterial: Streptococcus, Staphylococcus, Pseudomonas, Burkholderia, Mycobacterium, Chlamydophila, Ehrlichia, Rickettsia, Salmonella, Neisseria, Brucella, Mycobacterium, Nocardia, Listeria, Francisella, Legionella, and Yersinia
- Viral: Adenovirus, Herpes simplex, Varicella-zoster virus, Cytomegalovirus Papillomavirus, Hepatitis B virus Hepatitis C virus, Hepatitis E virus, Poliovirus, Yellow fever virus, Dengue virus, West Nile virus, TBE virus, HIV, Influenza virus, Lassa virus, Rotavirus and Ebola virus
- Fungal: Candida, Aspergillus, Cryptococcus, Histoplasma, Pneumocystis and Stachybotrys
- Parasites: protozoan parasites, helminth parasites and arthropod parasites

It is evident that the reliable detection and optionally quantification of a pathogen may be of high relevance in the diagnosis of the presence and severity of a disease.

The methods of the invention may be used in order to detect and quantify a specific cell, e.g. sub-population of cells. Examples of such cells include: cancer cells such as circulating tumor cells or circulating tumor microemboli, particular blood cells, such as B cells, T cells, eosinophils, etc. The cells may be rare cells, particularly wherein in the population the ratio of rare cells to total cells is at most 5%, preferably at most 1%, especially at most 0.1%, such as at most 0.01 %. Rare cells may be in particular circulating tumor cells (CTC) and circulating tumor microemboli (CTM) in a patient's blood. Finding and quantifying 'rare' tumor cells (just a few CTCs mixed with the approximately 10 million leukocytes and 5 billion erythrocytes in 1 ml of blood) and being able to distinguish them from other cells, particularly epithelial non-tumor cells and leukocytes is of particular relevance in the early detection of cancer. These cells may be detected long before the tumor itself is detectable, which is evidently highly advantageous in the treatment of the cancerous diseases.

Cancer cells are characterized by particular markers (tumor markers), which may be detected or quantified with the present methods. Examples which may be mentioned are: especially oncogenes and tumor suppressor genes such as p53, genes of the ras family erb-B2, c-myc, mdm2, c-fos, DPC4, FAP, nm23, RET, WT1, and the like, LOHs, for example with regard to p53, DCC, APC, Rb and the like and also BRCA1 and BRCA2 in hereditary tumors, microsatellite instability of MSH2, MLH1, WT1 and the like; also tumorous RNAs such as CEA, cytokeratins, e. g. CK20, BCL-2, MUC1, in particular tumor-specific splice variants hereof, MAGE3, Muc18, tyrosinase, PSA,PSM, BA46, Mage-1 and the like, or else morphogenic RNAs such as maspin, hCG, GIP, motilin, hTG, SCCA-1, AR, ER, PR, various hormones and the like ; -- furthermore, especially RNAs and proteins which affect the metastasizing profile, i. e. the expression of molecules involved in angiogenesis, motility, adhesion and matrix degradation such as bFGF, bFGF-R, VEGF, VEGF-Rs, such as VEGF-R1 or VEGF-R2, E-cadherin, integrins, selectins, MMPs, TIMPs, SF, SF-R and the like, the cell cycle profile or proliferation profile, such as cyclins (e. g. expression ratio of cyclins D, E and B), Ki67, p120, p21, PCNA and the like, or the apoptosis profile, such as FAS (L+R), TNF (L+R), perforin, granzyme B, BAX, bcl-2, caspase 3 and the like.

Alternative cells which may be determined by the methods of the invention include cardiovascular cells or vascular cells or vascular cells released by an inflammatory process or a fetal cell, e.g. a fetal cell in maternal blood, stem cells (e.g. cancerous stem cells), cells indicative of a minimal residual disease, cancer cells (e.g. leukemia cells). In this context, the method may be used for genotyping, diagnosis, prognosis, monitoring treatment etc.

The methods may also be used to detect and quantify the content of cells of a mammal species (e.g. in food control), a genetic status (e.g. when detecting or monitoring genetic disorders) or a disease.

In an eight aspect, the present invention relates to the use of the site-specifically conjugated antibody of the present invention (as defined above) in the detection of a target and/or in the diagnosis, particularly for the detection of a disease or a pathogen or in monitoring of a patient, especially in therapy monitoring or determining therapy efficiency of a disease. The definitions and comments with respect to the other aspects of the invention apply.

Unless defined otherwise, all technical and scientific terms and any acronyms used herein have the same meanings as commonly understood by one of ordinary skill in the art in the field of the invention. Definitions of common terms in molecular biology can be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1 -56081 -569-8).

The invention is not limited to the particular methodology, protocols, and reagents described herein because they may vary. Although any methods and materials similar or equivalent to those described herein can be used in the practice of the present invention, the preferred methods, and materials are described herein. Further, the terminology used herein is for the purpose of describing particular embodiments only and is not intended to limit the scope of the present invention.

As used herein and in the appended claims, the singular forms "a", "an", and "the" include plural reference unless the context clearly dictates otherwise. Similarly, the words "comprise", "contain" and "encompass" are to be interpreted inclusively rather than exclusively. Similarly, the word "or" is intended to include "and" unless the context clearly indicates otherwise. The term "plurality" refers to two or more.

The following Examples are intended to illustrate various embodiments of the invention. As such, the specific modifications discussed are not to be construed as limitations on the scope of the invention. It will be apparent to the person skilled in the art that various equivalents, changes, and modifications may be made without departing from the scope of the invention, and it is thus to be understood that such equivalent embodiments are to be included herein.

### FIGURES

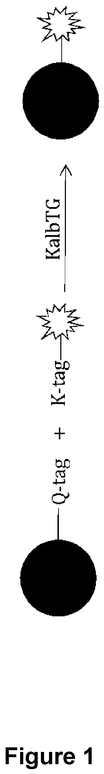

**Figure 1** illustrates a schematic overview of KalbRG enzymatic labelling reaction.
**Figure 2** illustrates a schematic overview of the IgG molecule showing the sites of Q-tag insertions with the promising candidates starred.
**Figure 3** illustrates illustrates the decision flow chart for the assessment of single-site Q-tagged-IgGs.
**Figure 4** illustrates the decision flow chart for the assessment of multi-site Q-tagged-IgGs.

### EXAMPLES

### Example 1: Identification of sites suitable for labelling

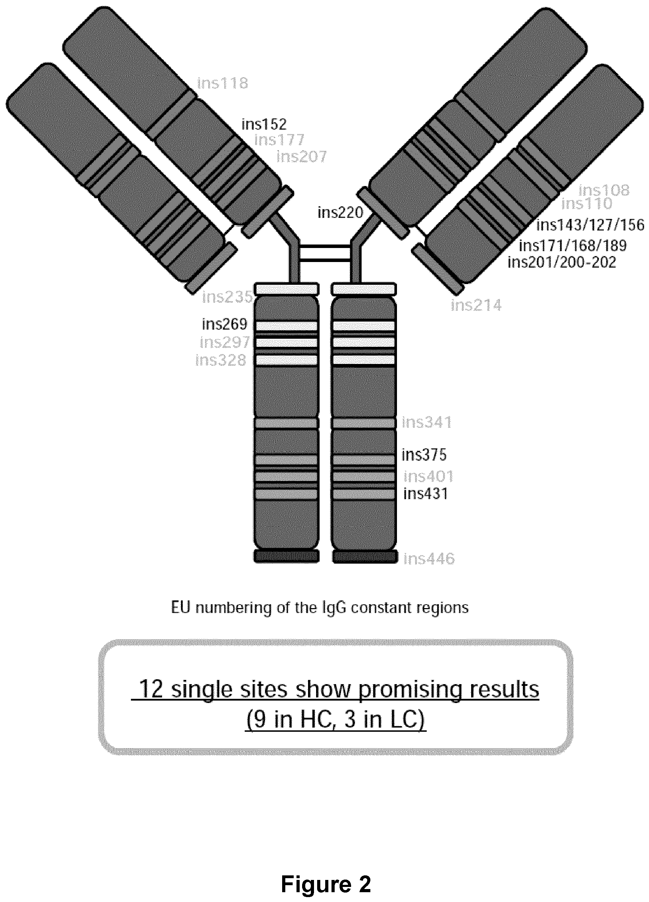

KalbTG Q-tags were inserted at surface-exposed inter- and intradomain flexible loops within the human IgG1 constant regions as well as the chains C termini; see Figure 2 for a schematic overwiew.

In total 24 distinct sites within the IgG heavy and light chains were selected to be tested; each with an insertion of the KalbTG Q-tag motif within two flexible linkers (GGGSYRYRQGGGS) (SEQ ID NO: 28) and in some instances without the flexible linker flankings (YRYRQ) (SEQ ID NO: 14).

The above sequence set (SEQ ID NO: 1 to 13) includes markings of the insertion sites within the human IgG1 heavy chain and the human light chain sequence as well as the homologous sites within the IgG heavy and light chain sequences of other species. The nucleic acid sequences encoding the modified IgGs described before were cloned into a standard mammalian cell expression vector with a leading extracellular secretion signal-sequence. The vectors were transfected into HEK293 cells and the modified IgG constructs were expressed following standard transient expression protocols. The modified IgG molecules were then purified from the cell culture supernatants using Protein A affinity chromatography.

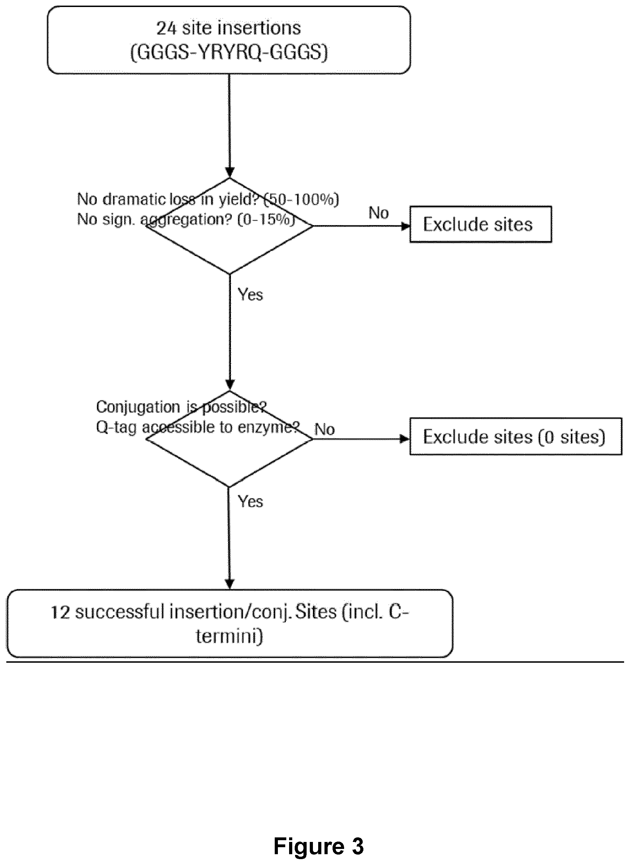

Purified IgG molecules with insertions at a single-site in either the heavy chain or the light chain were assessed for their expression rate (mg IgG per mL culture supernatant). In addition, successful folding and aggregation tendency were assessed via size-exclusion chromatography. Furthermore, the stability of the modified IgGs was tested using Dynamic Light Scattering (DLS) thermoscanning. Finally, the conjugation efficiency of the IgG molecules with single-site insertions (i.e. the success of the enzymatic conjugation reaction targeting these sites) was assessed by enzymatically labelling the IgGs with two different labels, in independent labelling experiments, under standard reaction conditions. Standard experimentation addressed two labels, Biotin (Bi) and sulfo-Ruthenium (sBPRu), both of them linked to a K-tag (RYESK) (SEQ ID NO: 21) via a PEG-based linker. See Table 1 and the respective legend for detailed overview on the results. In the first assessment phase, sites were selected based on advantageous expression yield and low tendency to aggregation. Sites without dramatic loss of expression yield (50-100%) and with ≤ 15% aggregates were selected for follow-up. Additionally, some sites which at least partially met the set criteria were also selected for follow-up (checkpoint 1). In the second assessment phase, sites were short-listed based on conjugation efficiency; sites which allowed for quantitative conjugation of the target indicating access of the Q-tag for the enzyme, were selected (checkpoint 2). See Figure 3 for an overview of the decision flow chart. Twelve successful internal and one C-terminal insertion/conjugation sites were identified.

Table 1 (attached below) provides a summary of the molecules (IgGs with single-site insertions) assessment data with more detailed description in the table legend.

**Table 1: Summary of data for single-site insertions**

| **Construct¹** | **Yield² (mg/L)** | **% Agg_{.}³** | **CP 1** | **Conjug. Eff.⁴** | **DLS⁵** | **CP 2** |
|---|---|---|---|---|---|---|
| **Heavy Chain Sites** | | | | | | |
| HC; 118(loop1) insGGGS-YRYRQ-GGGS | 150 | X | **Y** | Complete (Bi) 0.08 (sBPRu) | 56.56 | **Y** |
| HC; 118(loop1) insYRYRQ | 50 | 19% | **N** | None (Bi) 0.00 (sBPRu) | 56.84 | **N** |
| HC; 152 insGGGS-YRYRQ-GGGS | 41 | X | **N** | None (Bi) 0.01 (sBPRu) | 47.99 | **N** |
| HC; 177 insGGGS-YRYRQ-GGGS | 71 | X | **P** | Complete (Bi) 0.08 (sBPRu) | 59.84 | **Y** |
| HC; 207 insGGGS-YRYRQ-GGGS | 110 | X | **Y** | Complete (Bi) 0.07 (sBPRu) | 54.49 | **Y** |
| HC; 220(uphinge) insGGGS-YRYRQ-GGGS | 110 | X | **Y** | Incomplete (Bi) 0.04 (sBPRu) | 62.96 | **N** |
| HC; 235(hinge) insGGGS-YRYRQ-GGGS | 63 | 35% | **P** | Complete (Bi) 0.06 (sBPRu) | 62.96 | **Y** |
| HC; 269 insGGGS-YRYRQ-GGGS | 53 | 24% | **P** | Complete (Bi) 0.09 (sBPRu) | 62.31 | **Y** |
| HC; 297 insGGGS-YRYRQ-GGGS | 75 | 10% | **P** | Complete (Bi) 0.08 (sBPRu) | 62.78 | **Y** |
| HC; 328 insGGGS-YRYRQ-GGGS | 118 | X | **Y** | - 0.07 (sBPRu) | 60.14 | **Y** |
| HC; 341 (loop3) insGGGS-YRYRQ-GGGS | 180 | X | **Y** | Incomplete (Bi) 0.08 (sBPRu) | 61.35 | **Y** |
| HC; 375 insGGGS-YRYRQ-GGGS | 5 | 60% | **N** | Incomplete (Bi) | - | - |
| HC; 401 insGGGS-YRYRQ-GGGS | 104 | 8% | **Y** | Complete (Bi) 0.09 (sBPRu) | 64.10 | **Y** |
| HC; 431 insGGGS-YRYRQ-GGGS | 0 | X | **N** | - | - | - |

| **Light Chain Sites** | | | | | | |
|---|---|---|---|---|---|---|
| LC;110(loop1) insGGGS-YRYRQ-GGGS | 105 | X | **Y** | Incomplete (Bi) 0.07 (sBPRu) | 57.39 | **Y** |
| LC; 143 insGGGS-YRYRQ-GGGS | 16,5 | 18% | **N** | Complete (Bi) 0.08 (sBPRu) | 56.12 | **N** |
| LC; 171 insGGGS-YRYRQ-GGGS | 40 | X | **N** | Incomplete (Bi) 0.03 (sBPRu) | 59.28 | **N** |
| LC; 201 insGGGS-YRYRQ-GGGS | 22 | 27% | **N** | Complete (Bi) 0.09 (sBPRu) | 58.45 | **N** |
| LC; 108 insGGGS-YRYRQ-GGGS | 94 | 12% | **Y** | - | - | **Y** |
| LC; 127 insGGGS-YRYRQ-GGGS | 28 | 27% | **N** | - | - | - |
| LC; 156 insGGGS-YRYRQ-GGGS | 23 | 21% | **N** | - | - | - |
| LC; 168 insGGGS-YRYRQ-GGGS | 37 | 16% | **N** | - | - | - |
| LC; 189 insGGGS-YRYRQ-GGGS | 5 | 31% | **N** | - | - | - |
| LC; 214(Cterm) insGGGS-YRYRQ-GGGS | 155 | X | **Y** | Complete (Bi) 0.07 | 63.89 | **Y** |

| | | | | | | |
|---|---|---|---|---|---|---|
| **¹** for single-site insertions: constructs indicated by EU number of the amino acid (s) of the H-IgG1 constant regions after which the Q-tag motif is inserted (flanked by GGGS on each side or not; as indicated) **²** yield after purification and aggregates removal if any, wildtype clone (with no insertions) had an expression yield around 150 mg/ml **³** estimate of the percentage of aggregates (before their removal) from total expressed IgG, (x) in general refers to no or <10% aggregates **⁴** conjugation efficiency: For K-tag-biotin, judged by Streptavidin-Fluorescein interaction assay (Complete: complete conjugation (<5-10% unconjugated material), Incomplete: incomplete conjugation, None: no conjugation observed); For K-tag-sulfo-Ruthenium (sBPRu), judged by absorbance quotient at two wave lengths (A455/A480), A455/A280 ratio around 0,08-0,09 is expected for two sBPRu per IgG, a qualitative cut-off is picked for conjugation success assessment and conjugates categorization ⁵ Dynamic Light Scattering (DLS) thermoscanning, temperature at onset of hydrodynamic radius change by 0.2 nm - refers to experiment not done or data not available * refers to IgG that precipitated at high concentrations | | | | | | |

Under "Checkpoint 1" (CP1) and "Checkpoint 2" (CP") columns: Y (Yes), N (no) and P (Partially) refers to whether the construct passes the set criteria for the checkpoint as shown on the decision chart.

As shown in Table 1, the inventors have successfully identified a number of KalbTG Q-tag insertion sites spanning the length of the IgG backbone, which provide enzyme accessibility and do not adversely interfere with the IgG cellular expression or folding.

### Example 2: Identification of combination of sites allowing for multiple labelling

The single sites identified in Example 1 serve as the building blocks for the construction of an IgG backbone with multiple insertion motifs (Q-tags) for multi-site-specific conjugation via KalbTG. Moreover, they allow us to further explore the attachment site(s) effect on the label performance. Accordingly, IgG molecules, which contain 2, 3, 4, 5 or 6 combinations of the single insertions sites in Example 1, were tested.

The nucleic acid sequences encoding the modified IgGs described before were cloned into a standard mammalian cell expression vector with a leading extracellular secretion signal-sequence. The vectors were transfected into HEK293 cells and the modified IgG constructs were expressed following standard transient expression protocols. The modified IgG molecules were then purified from the cell culture supernatants using Protein A affinity chromatography.

Purified IgG molecules with insertions at a single-site in either the heavy chain or the light chain were assessed for their expression rate (mg IgG per ml culture supernatant). Successful folding and aggregation tendency were also assessed via size-exclusion chromatography. Finally, the conjugation efficiency of the IgGs with multiple site insertions (i.e. the success of the enzymatic conjugation reaction targeting these sites) was assessed by enzymatically labeling the IgGs with a K-tag-azide label; under standard reaction conditions. K-tag-azide is a hetero-bi-functional linker, where a K-tag (RYESK) (SEQ ID NO: 21) is linked through a PEG-like linker to an azide moiety. The azide group serves as a handle for a subsequent "Click"-reaction to attach any label/protein with the complementary reactive group (e.g. alkyne group). See Table 2 and its legend for detailed overview on the results.

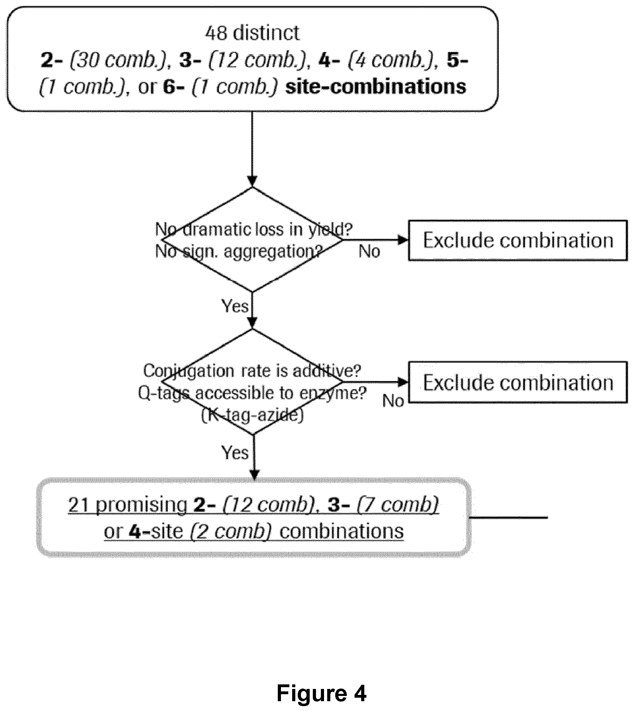

At checkpoint 1 (CP 1), sites were selected which did not result in dramatic loss of yield and which did not show significant aggregation. Other sites were excluded. At checkpoint 2 (CP2), sites were selected, which allowed for additive conjugation at four or more inserted KalbTG recognition sites within the target, and wherein the Q-tag was accessible for the enzyme. See Figure 4 for an overview of the decision flow chart in this regard. In the process, 21 surprisingly successful combinations were identified.

Table 2 (attached below) provides a summary of the molecules (IgGs with multiple-site insertions) assessment data with more detailed description in the table legend.

**Table 2: Summary of data for multiple-site insertions**

| **Construct¹** | **Yield² (mg/L)** | **%Agg.³** | **Checkpoint 1** | **Conjug. Eff.⁴** | **Checkpoint 2** |
|---|---|---|---|---|---|
| **Two Site Combinations** | | | | | |
| HC: 118; 341 insGGGS-YRYRQ-GGGS | 160 | X | **Y** | 3.74 | **Y** |
| HC: 118; 328 insGGGS-YRYRQ-GGGS | 120 | X | **Y** | 3.92 | **Y** |
| HC: 118; 401 insGGGS-YRYRQ-GGGS | 60 | X | **N** | - | - |
| HC: 207; 341 insGGGS-YRYRQ-GGGS | 160 | X | **Y** | 3.92 | **Y** |
| HC: 207; 328 insGGGS-YRYRQ-GGGS | 180 | X | **Y** | - | **P** |
| HC: 207; 401 insGGGS-YRYRQ-GGGS | 35 | X | **N** | 3.88 | **P** |
| HC: 177; 341 insGGGS-YRYRQ-GGGS | 97 | ? | **Y** | 3.83 | **Y** |
| HC: 177; 328 insGGGS-YRYRQ-GGGS | 36 | 9% | **N** | 3.8 | **P** |
| HC: 177; 401 insGGGS-YRYRQ-GGGS | 70 | 4% | **P** | 3.8 | **P** |
| HC: 328; 341 insGGGS-YRYRQ-GGGS | 13 | X | **N** | - | - |
| HC: 297; 341 insGGGS-YRYRQ-GGGS | 44 | 17% | **P** | - | - |
| HC: 341; 401 insGGGS-YRYRQ-GGGS | 20 | 15% | **N** | - | - |
| HC: 328; 401 insGGGS-YRYRQ-GGGS | 10 | 10% | **N** | - | - |
| HC: 297; 401 insGGGS-YRYRQ-GGGS | 25 | 11% | **N** | - | - |
| HC: 118; 177 insGGGS-YRYRQ-GGGS | 70 | X | **Y** | 3.91 | **Y** |
| HC: 118; 207 insGGGS-YRYRQ-GGGS | 2,7 | X | **N** | - | - |
| HC: 177; 207 insGGGS-YRYRQ-GGGS | 22 | X | **P** | 4.00 | **P** |
| HC: 328; 446 insGGGS-YRYRQ/RVRQR-GGGS | 69 | 11% | **Y** | 3.96 | **Y** |
| HC: 341;446 insGGGS-YRYRQ/RVRQR-GGGS | 100 | X | **Y** | 3.69 | **Y** |
| HC: 401; 446 insGGGS-YRYRQ/RVRQR-GGGS | 40 | 6% | **N** | 1.99 | **N** |
| HC: 118; LC: 214 insGGGS-YRYRQ-GGGS | 81 | X | **P** | 3.38 | **Y** |
| HC: 177; LC: 214 insGGGS-YRYRQ-GGGS | 66 | X | **P** | 3.65 | **Y** |
| HC: 207; LC: 214 insGGGS-YRYRQ-GGGS | 106 | X | **Y** | 3.64 | **Y** |
| HC: 118; LC: 110 insGGGS-YRYRQ-GGGS | 7 | 10% * | **N** | - | - |
| HC: 177; LC: 110 insGGGS-YRYRQ-GGGS | 25 | X | **N** | - | - |
| HC: 207; LC: 110 insGGGS-YRYRQ-GGGS | 1 | X | **N** | - | - |
| HC: 118; LC: 108 insGGGS-YRYRQ-GGGS | 52 | 8% * | **N** | - | - |
| HC: 177; LC: 108 insGGGS-YRYRQ-GGGS | 50 | X | **P** | 3.51 | **Y** |
| HC: 207; LC: 108 insGGGS-YRYRQ-GGGS | 2 | X | **N** | - | - |
| HC: 446; LC: 214 insGGGS-RVRQR-GGGS (C-termini) | 42 | | | | **Y** |

| **Three Site Combinations** | | | | | |
|---|---|---|---|---|---|
| HC: 118; 235; 341 insGGGS-YRYRQ-GGGS | 106 | X | **Y** | 5,08 | **Y** |
| HC: 207; 235; 341 insGGGS-YRYRQ-GGGS | 137 | X | **Y** | 5,92 | **Y** |
| HC: 177; 235; 341 insGGGS-YRYRQ-GGGS | 60 | X | **P** | 3,62 | **N** |
| HC: 118; 297; 341 insGGGS-YRYRQ-GGGS | 85 | X | **P** | 4 | **N** |
| HC: 207; 297; 341 insGGGS-YRYRQ-GGGS | 55 | X | **P** | 4,2 | **N** |
| HC: 177; 297; 341 insGGGS-YRYRQ-GGGS | 65 | X | **P** | 2,53 | **N** |
| HC: 118; 341; 446 insGGGS-YRYRQ/RVRQR-GGGS | 85 | | **Y** | 5.88 | **Y** |
| HC: 207; 341; 446 insGGGS-YRYRQ/RVRQR-GGGS | 78 | X | **Y** | 5,9 | **Y** |
| HC: 177; 341; 446 insGGGS-YRYRQ/RVRQR-GGGS | 23 | | **N** | - | - |
| HC: 118; 235; 328 insGGGS-YRYRQ-GGGS | 152 | 4% | **Y** | 5.8 | **Y** |
| HC: 207; 235; 328 insGGGS-YRYRQ-GGGS | 88 | 1% | **Y** | 5,72 | **Y** |
| HC: 177; 235; 328 insGGGS-YRYRQ-GGGS | 65 | X | **P** | 5,78 | **Y** |

| **Four Site Combinations** | | | | | |
|---|---|---|---|---|---|
| HC: 118; 235; 341; 446 insGGGS-YRYRQ/RVRQR-GGGS | 115 | X | **Y** | 7.85 | **Y** |
| HC: 207; 235; 341;446 insGGGS-YRYRQ/RVRQR-GGGS | 71,5 | X | **Y** | 7,9 | **Y** |
| HC: 207; 341; 446-RVRQR; LC: 214 YRYRQ/RVRQR | 1,85 | | **N** | - | - |
| HC: 207; 235; 341; LC: 214 insGGGS-RVRQR-GGGS | 10 | | **N** | - | - |

| **Five Site Combinations** | | | | | |
|---|---|---|---|---|---|
| HC: 207; 235; 341; 446-RVRQR LC: 214 YRYRQ/RVRQR | 2 | | **N** | - | - |

| **Six Site Combinations** | | | | | |
|---|---|---|---|---|---|
| HC: 207; 235; 297; 341; 446 LC: 214 insGGGS-RVRQR-GGGS | 0 | | **N** | - | - |

| | | | | | |
|---|---|---|---|---|---|
| **¹** for multiple-site-insertions: constructs indicated by EU number of the amino acid (s) of the H-IgG1 constant regions after which the Q-tag motif (YRYRQ) (SEQ ID No: 14) or an alternate Q-tag motif (RVRQR) (SEQ ID No:15) is inserted (flanked by GGGS on each side) as indicated. When indicated, RVRQR (SEQ ID NO: 15) are used for the C-terminal insertions. **²** yield after purification and aggregates removal if any, wildtype clone clone (with no insertions) has an expression yield around 150 mg/ml **³** estimate of the percentage of aggregates (before their removal) from total expressed IgG, (x) in general refers to no or <10% aggregates **⁴** conjugation efficiency: For K-tag-azide, by mass spectrometry incorporation determination - refers to experiment not done or data not available * refers to IgG that precipitated at high concentrations | | | | | |

Under "Checkpoint 1" (CP1) and "Checkpoint 2" (CP") columns: Y (Yes), N (no) and P (Partially) refers to whether the construct passes the set criteria for the checkpoint as shown on the decision chart (Figure 4).

As shown in Table 2, the inventors have successfully identified a number of IgG molecules with 2-, 3- and even 4- multiple-site-combinations, which allows the insertion of 4, 6 and 8 labels site-specifically and further enhance the ability to scan the site-dependence of label performance.

### REFERENCES

- Agarwal, P. & Bertozzi, C. R. Site-specific antibody-drug conjugates: the nexus of bioorthogonal chemistry, protein engineering, and drug development. Bioconjug Chem 26, 176-192, doi:10.1021/bc5004982 (2015).
- Ando, H. et al. Purification and Characteristics of a Novel Transglutaminase Derived from Microorganisms. Agricultural and Biological Chemistry 53, 2613-2617, doi:10.1080/00021369.1989.10869735 (2014).
- Chichili et al. Linkers in the structural biology of protein-protein interactions Protein Sci. 2013 Feb; 22(2): 153-167
- Steffen, W. et al. Discovery of a microbial transglutaminase enabling highly site-specific labelling of proteins. J Biol Chem, doi:10.1074/jbc.M117.797811 (2017).
- Strop, P. et al. Location matters: site of conjugation modulates stability and pharmacokinetics of antibody drug conjugates. Chem Biol 20, 161-167, doi:10.1016/j.chembiol.2013.01.010 (2013).
- WO 2016/100735
- WO 2017/102759
- WO 2021/174091
- Zhou, Q. & Kim, J. Advances in the Development of Site-Specific Antibody-Drug Conjugation. (2015).

## Claims

1. A modified antibody comprising a heavy chain and a light chain, wherein the antibody is modified to include, by way of insertion, one or more first recognition site(s) for the transglutaminase from *Kutzneria albida* (KalbTG), each insertion being located after one or more of the positions selected independently of each other from position 108 (LC108), position 110 (LC110) and position 214 (LC214) of the light chain, and position 118 (HC118), position 177 (HC177), position 207 (HC207), position 235 (HC235), position 269 (HC269), position 297 (HC297), position 328 (HC328), position 341 (HC341) and position 401 (HC401) of the heavy chain.

2. The modified antibody of claim 1 including a further first recognition site for KalbTG at position 446 (HC446) of the heavy chain.

3. The modified antibody of claim 1 or 2, wherein (i) the antibody comprises two pairs each of a heavy chain and a light chain, wherein each of the heavy chains or /and the light chains comprises the one or more first recognition sites, or (ii) the antibody comprises three or more pairs each of a heavy chain and a light chain, wherein each heavy chain is a fusion polypeptide comprising one or more additional Fab domain VH and CH1 heavy chain fragment fused to any of a VH N-terminus and Fc C-terminus of the heavy chain, wherein each of the fusion polypeptides or /and the light chains comprises the one or more first recognition sites, or (iii) the antibody comprises three or more pairs each of a heavy chain and a light chain, wherein each heavy chain is a fusion polypeptide comprising one or more additional Fab domain VL and CL light chain fragment fused to any of a VH N-terminus and Fc C-terminus of the heavy chain, wherein each fused Fab domain VL and CL light chain fragment is paired with a Fab domain VH and CH1 heavy chain fragment, wherein each of the fusion polypeptides or /and the light chains or /and the Fab domain VH and CH1 heavy chain fragment comprises the one or more first recognition sites.

4. The modified antibody of claim 1 to 3, wherein
a) the antibody includes two first recognition sites for KalbTG, particularly at positions
- HC118 / HC177; or
- HC118 / HC328; or
- HC118/HC341; or
- HC118 / HC401; or
- HC177 / HC207; or
- HC177 / HC328; or
- HC177 / HC341; or
- HC177 / HC401; or
- HC207 / HC328; or
- HC207 / HC341; or
- HC207 / HC401; or
- HC328 / HC446; or
- HC341 / HC446; or
- HC118 / LC214; or
- HC177 / LC108; or
- HC177 / LC214; or
- HC207 / LC214; or
- HC446 / LC214; especially
- HC118 / HC177; or
- HC118 / HC328; or
- HC118 / HC341; or
- HC177 / HC341; or
- HC207 / HC341; or
- HC328 / HC446; or
- HC341 / HC446; or
- HC118 / LC214; or
- HC177 / LC108; or
- HC177 / LC214; or
- HC207 / LC214; or
- HC446 / LC214;
OR
b) the antibody includes three first recognition sites for KalbTG, particularly at positions
- HC118 / HC235 / HC341; or
- HC207 / HC235 / HC341; or
- HC118 / HC341 / HC446; or
- HC207 / HC341 / HC446; or
- HC118 / HC235 / HC328; or
- HC177 / HC235 / HC328; or
- HC207 / HC235 / HC328;
OR
c) the antibody includes four first recognition sites for KalbTG, particularly at positions
- HC118 / HC235 / HC341 / HC446; or
- HC207 / HC235 / HC341 / HC446.

5. The modified antibody of any one of claims 1 to 4, wherein the one or more first recognition site(s) comprises or has a Gln-containing motif selected independently of each other from the group of sequences consisting of YRYRQ (SEQ ID NO: 14), YRQRT (SEQ ID NO: 16), RYGQR (SEQ ID NO: 17), RWRQR (SEQ ID NO: 18), RVRQR (SEQ ID NO: 15), IRQRQ (SEQ ID NO: 19) and FRYRQ (SEQ ID NO: 20), particularly YRYRQ (SEQ ID NO: 17) or RVRQR (SEQ ID NO: 18), especially YRYRQ (SEQ ID NO: 17).

6. The modified antibody of any one of claims 1 to 5, wherein the first recognition site or any one of two or more first recognition sites is linked to the antibody via one or two linker(s) at one or both termini of the respective first recognition site, especially wherein the linker consists primarily or fully of Gly and Ser, such as (Gly-Gly-Gly-Gly-Ser)ₙ (SEQ ID NO: 22) with n = 1, 2, 3, 4 or 5, preferably n=1, or especially wherein the linker comprises or consists of GGGP (SEQ ID NO: 23), ESGS (SEQ ID NO: 24) or APAP (SEQ ID NO: 25), wherein each of the two linkers at both termini is selected independently.

7. The modified antibody of any one of claims 1 to 6,
wherein the unmodified light chain constant domain (CL) comprises an amino acid sequence that is at least 96 %, 97 %, 98 %, or 99 %, particularly 100 % identical to the amino acid sequence of any of SEQ ID NO: 10 to 13; and/or wherein any of the unmodified heavy chain constant domains CH3, CH2, and CH1 comprises an amino acid sequence that is at least 96 %, 97 %, 98 %, or 99 %, particularly 100 % identical to the amino acid sequence of the respective domain in the amino acid sequence of SEQ ID NO: 1 to 9; and/or wherein the unmodified heavy chain constant domain comprises an amino acid sequence that is at least 96 %, 97 %, 98 %, or 99 %, particularly 100 % identical to the respective amino acid sequence of SEQ ID NO: 1 to 9; and/or
wherein the unmodified light chain constant domain (CL) consists of an amino acid sequence that is at least 96 %, 97 %, 98 %, or 99 %, particularly 100 % identical to the amino acid sequence of any of SEQ ID NO: 10 to 13; and/or wherein any of the unmodified heavy chain constant domains CH3, CH2, and CH1 consists of an amino acid sequence that is at least 96 %, 97 %, 98 %, or 99 %, particularly 100 % identical to the amino acid sequence of the respective domain in the amino acid sequence of SEQ ID NO: 1 to 9; and/or wherein the unmodified heavy chain constant domain consists of an amino acid sequence that is at least 96 %, 97 %, 98 %, or 99 %, particularly 100 % identical to the respective amino acid sequence of SEQ ID NO: 1 to 9.

8. The modified antibody of any one of claims 1 to 7, wherein the antibody
a) is a full antibody or one or more functional fragment(s) thereof, such as a single-chain Fv (scFv), a Fab fragment, a F(ab') fragment, a F(ab)2 fragment, a F(ab')2 fragment, an antibody/Fc fusion protein or a disulfide-linked Fvs (sdFv); and/or
b) is from goat, mouse, rat, rabbit or sheep; and/or
c) is human, humanized, chimeric and/or affinity matured.

9. A nucleic acid coding for a polypeptide chain of the modified antibody of any one of claims 1 to 8.

10. A site-specifically conjugated antibody, wherein the antibody comprises the modified antibody of any one of claims 1 to 8 and
(i) one or more labelling domain(s), wherein each of the one or more labelling domain(s) comprises a second recognition site for KalbTG or a functionally active variant thereof, wherein the second recognition site is covalently attached to a first recognition site of the modified antibody, and wherein the labelling domain comprises a detectable label; or
(ii) one or more chemical linker moiety /moieties, wherein each of the one or more covalently attached chemical linker moiety /moieties comprises a second recognition site for KalbTG or a functionally active variant thereof, wherein the second recognition site is covalently attached to a first recognition site of the antibody, and wherein the chemical linker moiety comprises a functional group capable of forming a covalent bond in a chemical (non-enzymatic) coupling reaction, particularly a functional group with a reaction partner of carbodiimide or N-hydroxy succinimide coupling chemistry, or a functional group with a reaction partner of so-called "click" chemistry, specifically a functional group with an azide or alkyne moiety.

11. The site-specifically conjugated antibody of claim 10, wherein to a chemical linker moiety a label is covalently attached, wherein the label is selected from any of a biotin moiety, a fluorescent dye, an iridium label, a ruthenium label, a radiolabel, a single-stranded oligonucleotide or analog thereof, and a chemiluminescent label.

12. The site-specifically conjugated antibody of claim 10 or 11, wherein
a) the label or the labelling domain is suitable for detection by means of an enzyme-mediated reaction, such as enzyme-mediated chromogenic, fluorogenic or metallographic reaction, or by means of direct or indirect fluorescence analysis or by means of radioanalysis or by means of electrochemoluminescence; and/or
b) the label or the labelling domain is or comprises an enzyme, an enzyme substrate, a chromophore, a fluorophore, a quencher, a radiolabel, a biotin, a metal, or an electrochemiluminescent moiety, specifically an electrochemiluminescent moiety comprising iridium or ruthenium; and/or
c) each site-specifically conjugated antibody is conjugated with at least 2, at least 4, at least 6 or at least 8 labels or labelling domains.

13. The site-specifically conjugated antibody of claim 11, wherein the linker is a mono or bi-functional chemical linker moiety permitting covalent attachment of one or two reactive further compounds, respectively.

14. A kit for producing a site-specifically conjugated antibody, the kit comprising the modified antibody of any one of claims 1 to 8 and at least one further component selected from the group consisting of
(i) KalbTG or a functionally active variant thereof;
(ii) a labelling domain capable of being bound to the one or more first recognition site(s) by KalbTG or a functionally active variant thereof, wherein the labelling domain comprises a detectable label; and
(iii) a chemical linker moiety capable of being bound to the one or more first recognition site(s) by KalbTG or the functionally active variant thereof, wherein the chemical linker moiety comprises a functional group functional group capable of forming a covalent bond in a chemical (non-enzymatic) coupling reaction, particularly a functional group with a reaction partner of carbodiimide or N-hydroxy succinimide coupling chemistry, or a functional group with a reaction partner of so-called "click" chemistry, specifically a functional group with an azide or alkyne moiety.

15. A method of site-specifically conjugating a modified antibody, the method comprising
a) providing the modified antibody of any one of claims 1 to 8,
b) providing a labelling domain, wherein the labelling domain comprises (i) a detectable label, (ii) a second recognition site for KalbTG, especially wherein the second recognition site comprises or has a motive with a primary amine group, more specifically a Lys-containing motif, especially the sequence RYESK (SEQ ID NO: 21), and (iii) optionally a linker between the label and the second recognition site; and
c) reacting the modified antibody of a) and the labelling domain of b) in the presence of KalbTG or a functionally active variant thereof and under conditions conducive to the activity of KalbTG, thereby forming a isopeptide bond between the first and the second recognition site, thus site-specifically conjugating the modified antibody;
or
a) providing the modified antibody of any one of claims 1 to 8,
b) providing a chemical linker moiety, wherein the chemical linker moiety comprises (i) a functional group functional group capable of forming a covalent bond in a chemical (non-enzymatic) coupling reaction, particularly a functional group with a reaction partner of carbodiimide or N-hydroxy succinimide coupling chemistry, or a functional group with a reaction partner of so-called "click" chemistry, specifically a functional group with an azide or alkyne moiety, and (ii) a second recognition site for KalbTG, especially wherein the second recognition site comprises or has a motive with a primary amine group, more specifically a Lys-containing motif, especially the sequence RYESK (SEQ ID NO: 21); and
c) reacting the modified antibody of a) and the chemical linker moiety of b) in the presence of KalbTG or a functionally active variant thereof and under conditions conducive to the activity of KalbTG, thereby forming a isopeptide bond between the first and the second recognition site, thus site-specifically conjugating the modified antibody.

16. Use of the modified antibody of any one of claims 1 to 8, KalbTG or a functionally active variant thereof, and (i) a second recognition site for KalbTG coupled to a detectable label or (ii) a second recognition site for KalbTG coupled to a chemical linker moiety which comprises a functional group capable of forming a covalent bond in a chemical coupling reaction, for producing a site-specifically conjugated antibody.

17. A method of detecting a target in a sample, the method comprising
a) contacting the sample, suspected of comprising the target, with the site-specifically conjugated antibody of any of claims 10 to 13, wherein the site-specifically conjugated antibody is capable of specifically binding to the target, under conditions conducive to the binding of the antibody to the target;
b) optionally removing unbound site-specifically conjugated antibody; and
c) detecting the label of the site-specifically conjugated antibody specifically bound to the target, thereby detecting the target.

18. The method of claim of claim 17,
a) wherein the target is a protein, such as a protein indicative of a microorganism, a cell, especially a tumor cell, a virus, a bacterium, a fungus, a mammal species, a genetic status, a physiological status or a disease, or another antibody; and/or
b) wherein the sample has been obtained from a source suspected of being contaminated, a cell culture or a subject, particularly wherein the subject is selected from the group consisting of a human, an animal and a plant, especially a human; and/or
c) wherein the sample is selected from the group consisting of a body fluid, blood, blood plasma, blood serum, urine, bile, cerebrospinal fluid, synovial fluid, a swab, a clinical specimen, an organ sample and a tissue sample.

19. Use of the site-specifically conjugated antibody of any of claims 10 to 13 in the detection of a target and/or in the diagnosis, particularly for the detection of a disease or a pathogen or in monitoring of a patient, especially in therapy monitoring or determining therapy efficiency of a disease.
